# EUROPEAN PATENT SPECIFICATION

(11) **EP 3 458 143 B1**
(45) Date of publication and mention of the grant of the patent: **01.02.2023**
(21) Application number: 17724885.3
(22) Date of filing: 15.05.2017
(51) Int. Cl.: A61M 39/10, A61M 39/20

(54) **IMPROVED FLUID LINE CONNECTOR DEVICE**
VERBESSERTE FLUIDLEITUNGVERBINDERVORRICHTUNG
DISPOSITIF DU TYPE RACCORD DE CONDUITE DE FLUIDE AMÉLIORÉ

(30) Priority: 16.05.2016 GB 201608603; 05.01.2017 GB 201700096
(43) Date of publication of application: 27.03.2019
(73) Proprietor: Medical Device Creations Limited, Mossley Hill, Liverpool L18 2DH (GB)
(72) Inventor: JEFFREY, Peter, LIVERPOOL Merseyside L19 9BJ (GB)
(74) Representative: HGF
(86) International application number: PCT/GB2017/051357
(87) International publication number: WO 2017/199012

(56) References cited:
- EP-B1- 2 051 770
- WO-A1-2005/079907
- US-A- 6 003 556
- US-A1- 2005 040 648
- US-B1- 6 508 807

## Description

This invention relates to an improved fluid line connector device for use in connecting two fluid lines together to form a fluid connection. Particularly, this invention relates to a one-piece fluid line connector having means for preventing a non-corresponding fluid line connector from connecting to the one-piece fluid line connector.

### BACKGROUND

Luer lock connectors are well known within the medical industry for connecting a fluid container to a fluid feed line e.g. connecting a syringe to an IV line. The connection consists of a male connector and a female connector engaging to provide a fluid tight seal. The male connector typically includes a luer taper configured to be received by the female connector. The male connector often includes a thread which matches lugs of the female connector configured to lock the two connectors together. The industry standard for lugs of the female connector is such that there are two lugs positioned opposite one another around the circumference of the female connector. It is known that non-standard luer lock connectors may be used in situations in which it is very important not to connect certain fluid feed lines to the certain fluid containers.

EP 2 051 770 B1 describes a connector system including a male connector, a female connector and a barrier configured to allow a correspondingly-shaped female connector to pass therethrough and engage a luer taper and a thread of the male connector. The female connector includes lugs spaced circumferentially around the female connector in a configuration different to the industry standard described above. The barrier of the male connector has a slot shaped to match the circumferential spacing of the lugs of the female connector. Typically, the barrier of EP 2 051 770 B1 is manufactured separate to the male connector and subsequently fastened to the male connector. Typically, the male connector and barrier are mass manufactured, and it may be time consuming for the user or a manufacturer to fasten the barrier onto the male connector, as the two parts are relatively small and difficult to handle. When fastened to the male connector, the barrier may rotate relative to the male connector. Consequently, it may be time consuming for a user to insert the female connector into a male connector as the slot of the barrier may not be aligned such that the female luer lock fitting engages the thread without suitable rotation of the barrier relative to the male connector. Further, upon removal of the female connector from the male connector, the barrier may not be aligned such that upon exiting the thread of the male connector, the female connector must be rotated further to exit the barrier. Furthermore, upon removal, the barrier may rotate with the female connector and the female lock fitting may become jammed within the barrier. Due to the often time-critical nature of using such luer lock connectors, it is desirable to have a system whereby the time taken to connect a male connector to a female connector is minimal and the risk of jamming is reduced.

In certain prior art arrangements, the barrier may inadvertently allow a non-correspondingly-shaped female connector to connect to the male connector. This may be achievable by inserting the non-correspondingly-shaped female connector through the slot of the barrier at an angle such that the lugs of the non-correspondingly-shaped female connector engage the thread of the male connector. It is not desirable to have a system which may possibly allow such incorrect connections. US 6508807 B1 describes a medical coupling for a cannulae including: a female member with a body providing a hub defining a socket; a valve actuator slidable on the body; and a male member including a spiget for engagement in the socket, and a cap with a screw thread for engagement with the hub. WO 2005/079907 A1 describes an infusion and/or withdrawal fitting for biomedical fluid circuits defined by a tubular conduit having a first end connectable to a biomedical fluid circuit, and a second end which is closed by a plug having a collar connected to the plug at a weakened portion.

It is an object of certain embodiments of the present invention to address the above-described disadvantages associated with the prior art.

### BRIEF SUMMARY OF THE DISCLOSURE

According to a first aspect of the invention, there is provided a one-piece fluid line connector for connecting two fluid lines together to form a fluid connection comprising:
a main body having a male luer lock fitting; and
a cap coupled to the main body by a living hinge;
wherein the living hinge is configured such that the cap is moveable to an aligned position in which the cap is aligned with a central axis of the male luer lock fitting of the main body, and
wherein the cap includes a through-hole configured such that, in the aligned position, the shape of the through-hole permits a correspondingly-shaped female luer lock fitting to pass therethrough and engage the male luer lock fitting.

In certain embodiments, the male luer lock fitting may include a thread and a luer taper, wherein the luer taper is configured to be received by a correspondingly-shaped female luer lock fitting and the thread is configured to receive one or more lugs of the correspondingly-shaped luer lock fitting.

In certain embodiments, the cap may have a guide portion configured to determine a helical movement of at least one of the one or more lugs between the cap and the thread when the female luer lock fitting is rotated in the cap, the at least one of the one or more lugs is adjacent the guide portion of the cap, and the cap is in the aligned position. The guide portion may comprise one or more first sloped surfaces configured to at least partially determine the helical movement of at least one of the one or more lugs. The at least a portion of the one or more first sloped surfaces may define a portion of the lowermost surface of the cap, wherein the lowermost surface of the cap faces the thread when the cap is in the aligned position. When the cap is in the aligned position, at least one of the one or more first sloped surfaces may cooperate with the thread to axially constrain at least one of the one or more lugs in at least one rotational position of the female luer lock fitting within the cap.

In certain embodiments, the guide portion may further comprise one or more second sloped surfaces facing in a direction opposite that of the first sloped surfaces, the one or more second sloped surfaces being configured to determine the helical movement of at least one of the one or more lugs between the thread and the cap when the cap is in the aligned position. At least a portion of the one or more second sloped surfaces may define a portion of the uppermost surface of the cap, wherein the uppermost surface of the cap is configured to face away from the thread when the cap is in the aligned position. At least a portion of the one or more second sloped surfaces may cooperate with the one or more first sloped surfaces to axially constrain at least one of the one or more lugs in at least one rotational position of the female luer lock fitting within the cap.

In certain embodiments, the one-piece fluid line connector may be arranged such that, when the cap is in the aligned position, in at least one rotational position of the female luer lock fitting within the cap all of the one or more lugs are axially restrained by one or more of the first sloped surfaces, the second sloped surfaces and the thread.

In certain embodiments, the through-hole may be configured to rotationally restrain at least one lug of a correspondingly-shaped female luer lock fitting in at least one axial position of the female luer lock fitting within the cap.

In certain embodiments, the main body may include the thread of the male luer lock fitting.

In certain embodiments, when the cap is in the aligned position, the cap may be fastened to the main body of the one-piece fluid line connector, or, the cap may be fastened to the male luer lock fitting of the main body. The cap may be fastened using a snap-fit connection. Additionally, the cap may be rotationally fixed relative to the main body.

In certain embodiments, the main body may include a portion configured to receive the cap when the cap is in the aligned position, such that the portion of the main body is radially external to the cap. The portion of the main body configured to receive the cap may be a portion of the male luer lock fitting of the main body, such that when the cap is in the aligned position, the portion of the male luer lock fitting is radially external to the cap. Additionally, the portion of the main body may include a flange and the cap may include a recess configured to engage with the flange of the main body such that the cap forms a snap-fit connection with the main body.

In certain embodiments, the cap may include a portion configured to receive a portion of the main body when the cap is in the aligned position, such that the portion of the cap is radially external to the main body. The portion of the main body received by the portion of the cap may be a portion of the male luer lock fitting of the main body such that, when the cap is in the aligned position, the portion of the cap is radially external to the male luer lock fitting of the main body. Additionally, the portion of the cap may include a flange and the portion of the main body may include a recess configured to engage with the flange of the cap such that the cap forms a snap-fit connection with the main body.

In certain embodiments, the through-hole of the cap may be configured such that, when the cap is in the aligned position, as a lug of a correspondingly-shaped female luer lock fitting may pass therethrough, the lug is aligned with an entrance of the thread of the male luer lock fitting. The through-hole of the cap may have a variable radius. The variable radius may comprise one or more adjacent sectors with different radii. At least one of the one or more adjacent sectors may have a shape configured to receive a correspondingly-shaped lug of a female luer lock fitting such to allow the correspondingly-shaped lug to pass therethrough.

In certain embodiments, the cap may comprise two or more radially inwardly extending portions to define the shape of the through-hole, wherein a circumferential spacing exists between each radially inwardly extending portion, each circumferential spacing defining a slot which determines the at least one of the one or more adjacent sectors having a shape configured to receive a correspondingly-shaped lug of a female luer lock fitting to allow the correspondingly-shaped lug to pass therethrough. At least one slot may have a different angular extent around the circumference of the cap than another slot. Each slot may have a different angular extent around the circumference of the cap.

In certain embodiments, the two or more radially inwardly extending portions may comprise a first radial portion, a second radial portion circumferentially spaced about the cap from the first radial portion, and a third radial portion circumferentially spaced about the cap from both the second radial portion and the first radial portion, wherein the circumferential spacing between the first radial portion and the second radial portion determines a first slot of the through-hole configured to receive a correspondingly-shaped lug of a female luer lock fitting, the circumferential spacing between the second radial portion and the third radial portion determines a second slot of the through-hole configured to receive a correspondingly-shaped lug of the female luer lock fitting, and the circumferential spacing between the third radial portion and the first radial portion determines a third slot of the through-hole configured to receive a correspondingly-shaped lug of the female luer lock fitting.

In certain embodiments, one of the first slot, the second slot and the third slot may have an angular extent of 20 degrees, one of the first slot, the second slot and the third slot may have an angular extent of 40 degrees, and one of the first slot, the second slot and the third slot may have an angular extent of 60 degrees.

In certain embodiments, each of the two or more radially inwardly extending portions may have a first sidewall configured to prevent rotation of a female luer lock fitting in a first rotational direction when a lug of the female luer lock fitting is adjacent thereto, and a second sidewall configured to prevent rotation of a female luer lock fitting in a rotational direction opposite the first rotational direction when a lug of the female luer lock fitting is adjacent thereto.

In certain embodiments, the shape of the two or more radially inwardly extending portions may define the guide portion.

In certain embodiments, when the cap is in the aligned position, the cap may be adjacent to the thread.

In certain embodiments, the main body may further comprise a conduit configured to be attached to a fluid feed container to provide a fluid connection between the fluid feed container and the male luer lock fitting of the main body.

In certain embodiments, when the cap is in the aligned position, the one-piece fluid line connector may further comprise a void positioned between the thread of the male luer lock fitting and the through-hole of the cap, the void configured to receive lugs of a correspond female luer lock fitting and permit free rotation of the female luer lock fitting when the lugs are received by the void. The cap may further comprise a stopper configured to partially prevent counter-clockwise rotation of a corresponding female luer lock fitting when lugs of the corresponding female luer lock fitting are positioned within the void.

In certain embodiments, the one-piece fluid line connector may comprise indicator means configured to aid the user in aligning lugs of a female luer lock fitting with the through-hole of the one-piece fluid line connector. The indicator means may comprise a tactile indicator. The tactile indicator may be positioned on the cap.

In embodiments of the invention, the cap may be rotationally fixed relative to the main body by means other than a living hinge.

According to a second aspect of the invention, there is provided a fluid line connector comprising:
a main body having a male luer lock fitting, the male luer lock fitting including a thread and a luer taper; and
a cap having a through-hole and a guide portion, the cap being rotationally fixed relative to the main body and the through-hole being configured to permit a correspondingly-shaped female luer lock fitting to pass therethrough and engage the male luer lock fitting,
wherein the luer taper is configured to be received by the correspondingly-shaped female luer lock fitting, the thread is configured to receive one or more lugs of the correspondingly-shaped female luer lock fitting, and the guide portion is configured to determine a helical movement of at least one of the one or more lugs between the cap and the thread when the female luer lock fitting is rotated in the cap and the at least one of the one or more lugs is adjacent the guide portion of the cap.

According to a third aspect of the invention, there is provided a fluid line connector system comprising:
a male luer lock fitting having a luer taper and a thread;
a female luer lock fitting having a connector and one or more lugs, the connector configured to receive the luer taper of the male luer lock fitting and the one or more lugs configured to be received by the thread of the male luer lock fitting; and
a cap rotationally fixable to the main body, the cap including a through hole configured such that, when the cap is fixed to the male luer lock fitting, the shape of the through hole permits the one or more lugs of the female luer lock fitting to pass therethrough and engage the internal thread of the collar of the main body;
wherein the fluid line connector system is configured such that, when the cap is rotationally fixed to the main body, the fluid connector system includes a void between the through hole of the cap and the thread of the male luer lock fitting, the void being configured to receive all of the one or more lugs of the female luer lock fitting.

In certain embodiments, at least one of the one or more lugs of the female luer lock fitting may be in a plane axially offset from a plane of a different lug.

In certain embodiments, at least one of the one or more lugs may be circumferentially spaced from a different lug.

In certain embodiments, the through hole of the cap may have a variable radius. The variable radius may comprise one or more adjacent sectors with different radii. At least one of the one or more adjacent sectors may have a radius configured to receive one of the one or more lugs of the female luer lock fitting such to allow the lug to pass therethrough. When the cap is in the aligned position, the one or more adjacent sectors of the through hole may be aligned with the thread of the male luer lock fitting such that a lug passing therethrough is aligned to engage the thread of the male luer lock fitting.

In certain embodiments, the through hole of the cap may include an internal surface substantially parallel to a central axis of the male luer lock fitting.

In certain embodiments, the void of the fluid line connector system may be of the order of 2 to 2.5mm in axial depth.

In certain embodiments, the male luer lock fitting may be attachable to a fluid feed container.

In certain embodiments, the female luer lock fitting is attachable to a fluid feed line.

In certain embodiments, the fluid line connector system may comprise indicator means configured to aid the user in aligning the lugs of the female luer lock fitting with the through-hole of the cap. The indicator means may comprise a tactile indicator on one or more of the female luer lock fitting, the male luer lock fitting and the cap.

According to a fourth aspect of the invention, there is provided a fluid line assembly comprising:
a one-piece fluid line connector according to the first aspect of the invention or a fluid line connector according to the second aspect of the invention; and
a female luer lock fitting connectable to the fluid line connector, the female luer lock fitting comprising one or more lugs configured to be received by the thread of the male luer lock fitting of the fluid line connector;
wherein the female luer lock fitting is configured to connect with the fluid line connector such that a fluid may flow between the main body of the fluid line connector and the female luer lock fitting.

In certain embodiments, one of the one or more lugs of the female luer lock fitting may be in a plane that is axially offset from a plane of a different one of the one or more lugs. The one or more lugs of the female luer lock fitting may be circumferentially spaced from each other. At least one of the one or more lugs may have a greater angular extent around the circumference of the female luer lock fitting than another of the one or more lugs.

In certain embodiments, the female luer lock fitting may comprise three lugs, wherein each of the three lugs is configured to be received by the thread of the male luer lock fitting of the fluid line connector. One of the three lugs may have a greater angular extent around the circumference of the female luer lock fitting than the other two of the three lugs. Each of the three lugs may have a different angular extent around the circumference of the female luer lock fitting than another of the three lugs. One of the three lugs may have an angular extent of 20 degrees, one of the three lugs may have an angular extent of 40 degrees, and one of the three lugs may have an angular extent of 60 degrees.

### BRIEF DESCRIPTION OF THE DRAWINGS

Embodiments of the invention are further described hereinafter with reference to the accompanying drawings, in which:
Figure 1A shows a one-piece fluid line connector in accordance with an embodiment of the present invention;
Figure 1B shows the one-piece fluid line connector of Figure 1A with the cap disposed in the main body of the one-piece fluid line connector;
Figure 2A is a cross-sectional view of the one-piece fluid line connector of Figure 1A;
Figure 2B is a cross-sectional view corresponding to Figure 1B;
Figure 3A shows a one-piece fluid line connector in accordance with another embodiment of the present invention;
Figure 3B shows the one-piece fluid line connector of Figure 3A with a portion of the main body received by the cap of the one-piece fluid line connector;
Figure 4A is a cross-sectional view of the one-piece fluid line connector of Figure 3A;
Figure 4B is a cross-sectional view corresponding to Figure 3B;
Figure 5A shows an example female luer lock fitting suitable for connecting with a one-piece fluid line connector according to an embodiment of the present invention;
Figure 5B is a cross-sectional view of the female luer lock fitting of Figure 5A;
Figure 6A is a schematic view showing the circumferential distribution of the lugs of the female luer lock fitting of Figure 5A;
Figure 6B is a top view of a cap of a one-piece fluid line connector according to an embodiment of the present invention;
Figure 6C is a schematic view of the female luer lock fitting of Figure 6A received by the cap of Figure 6B;
Figure 7A is a schematic view showing the circumferential distribution of the lugs of another example female luer lock fitting;
Figure 7B is a top view of a cap of a one-piece fluid line connector according to another embodiment of the present invention;
Figure 7C is a schematic view of the female luer lock fitting of Figure 7A received by the cap of Figure 7B;
Figure 8A is a schematic view showing the circumferential distribution of the lugs of another example female luer lock fitting;
Figure 8B is a top view of a cap of a one-piece fluid line connector according to another embodiment of the present invention;
Figure 8C is a schematic view of the female luer lock fitting of Figure 8A received by the cap of Figure 8B;
Figure 9A is a schematic view showing the circumferential distribution of the lugs of another example female luer lock fitting;
Figure 9B is a top view of a cap of a one-piece fluid line connector according to another embodiment of the present invention;
Figure 9C is a schematic view of the female luer lock fitting of Figure 9A received by the cap of Figure 9B;
Figure 10A is a schematic overview of the spatial relationship between lugs of a female luer lock fitting and a through-hole of a one-piece fluid line connector according to an embodiment of the present invention;
Figure 10B is a schematic overview of the arrangement shown in Figure 10A in a partially connected configuration;
Figure 10C is a schematic overview of the relationship between the arrangement shown in Figure 10A in a pre-threaded configuration and a thread of a one-piece fluid line connector according to an embodiment of the present invention;
Figure 11 is a cross-sectional view of a one-piece fluid line connector with the cap disposed in the main body of the one-piece fluid line connector in accordance with an embodiment of the present invention;
Figure 12A is a schematic view showing the circumferential distribution of the lugs of another example female luer lock fitting;
Figure 12B is a top view of a cap of a one-piece fluid line connector according to another embodiment of the present invention;
Figure 12C is a schematic view of the female luer lock fitting of Figure 12A received by the cap of Figure 12B;
Figure 13A is a schematic overview of the spatial relationship between the lugs of a female luer lock fitting, and a thread and a through-hole of a one-piece fluid line connector in accordance with an embodiment of the present invention;
Figure 13B is a schematic overview of the arrangement shown in Figure 13A in an intermediate configuration;
Figure 13C is a schematic overview of the arrangement shown in Figure 13A in a threaded configuration;
Figure 14 is a cross-sectional view of a main body of a one-piece fluid line connector according to another embodiment of the present invention;
Figure 15A is a schematic top view of a through-hole of a cap of the one-piece fluid line connector of Figure 14;
Figure 15B is a bottom view of the cap of Figure 15A;
Figure 15C is a cross-sectional view along line a-a of Figure 15A;
Figure 15D is a cross-sectional view along line b-b of Figure 15A;
Figure 16A is a schematic overview showing the relationship between the main body according to Figure 14, the cap according to Figures 15A to 15D in an aligned position, and an example female luer lock fitting;
Figure 16B is a schematic overview of the arrangement shown in Figure 16A in a pre-threaded configuration; and
Figure 17 is a cross-sectional view of a one-piece fluid line connector with the cap disposed in the main body of the one-piece fluid line connector in accordance with another embodiment of the present invention.

### DETAILED DESCRIPTION

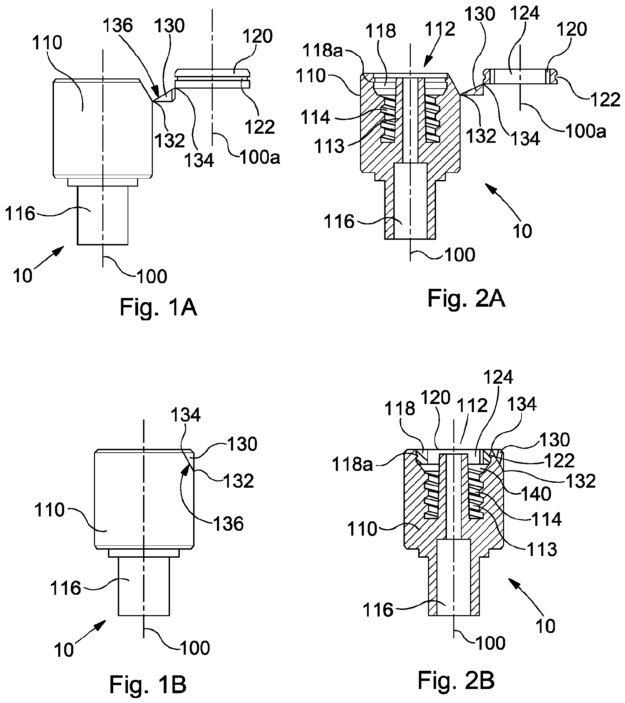

A one-piece fluid line connector 10 in accordance with an embodiment of the present invention is shown in Figure 1A. The one-piece fluid line connector 10 includes a main body 110 having a central axis 100, and a cap 120 coupled to the main body 110 by a living hinge 130. Figure 1B shows the one-piece fluid line connector 10 of Figure 1A in an aligned configuration, in which the cap 120 is aligned with the central axis 100 of the main body 110. That is, in the aligned configuration, a longitudinal axis 100a of the cap 120 is co-axial with the central axis 100 of the main body 110.

Figure 2A is a cross-sectional view of the one-piece fluid line connector 10 of Figure 1A. The main body 110 includes a male luer lock fitting 112 having a luer taper 113 and a thread 114, a conduit 116 in fluid communication with the male luer lock fitting 112, and a cap receiving portion 118 having a flange 118a around the circumference of the receiving portion 118. The cap 120 includes a recess 122 arranged circumferentially around the outside of the cap 120, and a through-hole 124 arranged through the centre of the cap 120. In certain embodiments, the cap receiving portion 118 may include a recess and the cap 120 may include a flange.

Figure 2B is a cross-sectional view of the one-piece fluid line connector 10 of Figure 1B. In the aligned configuration as shown in Figure 2B, the cap 120 is received by the cap receiving portion 118 of the main body 110.

The living hinge 130 is attached at a first end 132 to the main body 110 and at a second end 134 to the cap 120. The living hinge 130 is pivotable about both the first end 132 and the second end 134 so as to move the cap 120 from the position shown in Figures 1A and 2A, to the aligned position as shown in Figures 1 B and 2B. The living hinge 130 also has an inside surface 136 configured to abut against the main body 110 when in the aligned configuration shown in Figures 1 B and 2B. The inside surface 136 is configured to guide the movement of the cap 120 to align the longitudinal axis 100a of the cap 120 with the central axis 100 of the main body 110. The living hinge 130 is advantageous in that the one-piece fluid line connector 10 may be assembled simply by pivoting the living hinge 130 such that the cap 120 is received by the main body 110. In contrast, prior art methods of manufacturing non-standard fluid line connectors may involve assembling two separate parts together (e.g. a main body and a cap). For high volume manufacture, a small time saving per unit may be highly advantageous. In certain embodiments, the cap 120 may be coupled to the main body 110 of the one-piece fluid line connector 10 by any means achievable when manufactured from one-piece, so long as the cap 120 is moveable to the aligned position. For example, the cap 120 may be coupled to the main body 110 using a flexible strip.

By providing a coupling between the cap and the main body, the risk that a user may undesirably assemble an independent cap to an incorrect main body is eliminated.

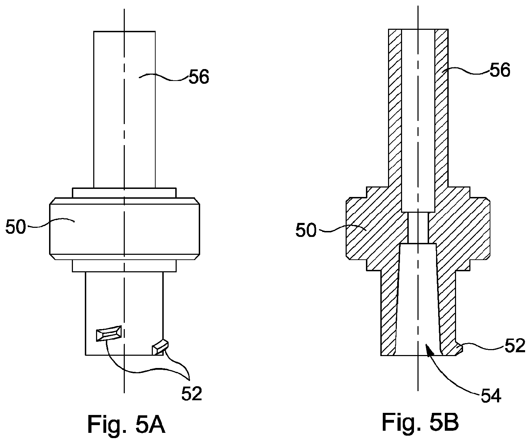

Figure 5A shows an example female luer lock fitting 50 suitable for connecting to the one-piece fluid line connector 10 of Figures 1A to 2B. Figure 5B is a cross-sectional view of the female luer lock fitting 50 of Figure 5A. The female luer lock fitting 50 includes circumferentially and axially spaced lugs 52 configured to be received by the thread 114 of the male luer lock fitting 112, a conically-shaped receiving portion 54 configured to receive the luer taper 113 of the male luer lock fitting 112, and a connector 56 configured to attach the female luer lock fitting 50 to a fluid feed line (not shown).

In other examples, the connector of the female luer lock fitting according to embodiments of the invention may be configured to attach to a standardised female luer lock fitting. This enables the standardised female luer lock fitting to be converted so that it may connect with a one-piece fluid line connector according to the present invention.

In use, the luer taper 113 of the male luer lock fitting 112 is received by the conically-shaped receiving portion 54 of the female luer lock fitting 50 to provide a fluid-tight connection between the one-piece fluid line connector 10 and the female luer lock fitting 50. The thread 114 of the male luer lock fitting 112 receives the lugs 52 of the female luer lock fitting 50 to provide a means for preventing the female luer lock fitting 50 from disconnecting during use. The conduit 116 of the main body 110 is configured to attach the main body 110 to a fluid feed container (not shown) such to permit a fluid connection between the fluid feed container (not shown) and the male luer lock fitting 112 of the main body 110. Consequently, in use, the main body 110 permits fluid flow between a fluid feed container (not shown) and a fluid feed line (not shown) via the female luer lock fitting 50.

In other embodiments, the conduit of the main body may be configured to attach to a standardised male luer lock fitting so that the one-piece fluid line connector may be used to convert a standardised male luer lock fitting to one which may only connect with a female luer lock fitting suitable for connecting with the one piece fluid line connector according to the present invention.

The cap receiving portion 118 of the main body 110 is configured such that, in the aligned configuration, the cap receiving portion 118 is radially external of the cap 120 as shown in Figure 2B. In the aligned configuration as shown in Figure 2B, the flange 118a may engage with the recess 122 of the cap 120 to form a snap-fit fastening between the cap 120 and the cap receiving portion 118 of the main body 110. The snap-fit fastening ensures that the cap 120 remains received by the cap receiving portion 118 during normal use of the one-piece fluid line connector 10. In certain embodiments, it is intended that the cap 120 will not be removed from the main body 110 once it is fastened to the main body 110.

The through-hole 124 of the cap 120 is configured such that, in the aligned configuration, the shape of the through-hole 124 permits a correspondingly-shaped female luer lock fitting to pass therethrough and engage the male luer lock fitting 112 of the main body 110. Conversely, a non-correspondingly-shaped female luer lock fitting will not be capable of passing through the through-hole 124, thus reducing the possibility of inadvertently connecting a fluid line connector to an incorrect fluid feed or outlet. An advantage of the cap receiving portion 118 being radially external of the cap 120 in the aligned configuration is that it may be difficult for a user to grip and remove the cap 120 once it is fastened to the cap receiving portion 118. This reduces the risk that a user may remove the cap 120 (thus removing the through-hole 124 that prevents a non-correspondingly-shaped female luer lock fitting from passing therethrough) to subsequently connect a non-correspondingly-shaped female luer lock fitting to the male luer lock fitting 112.

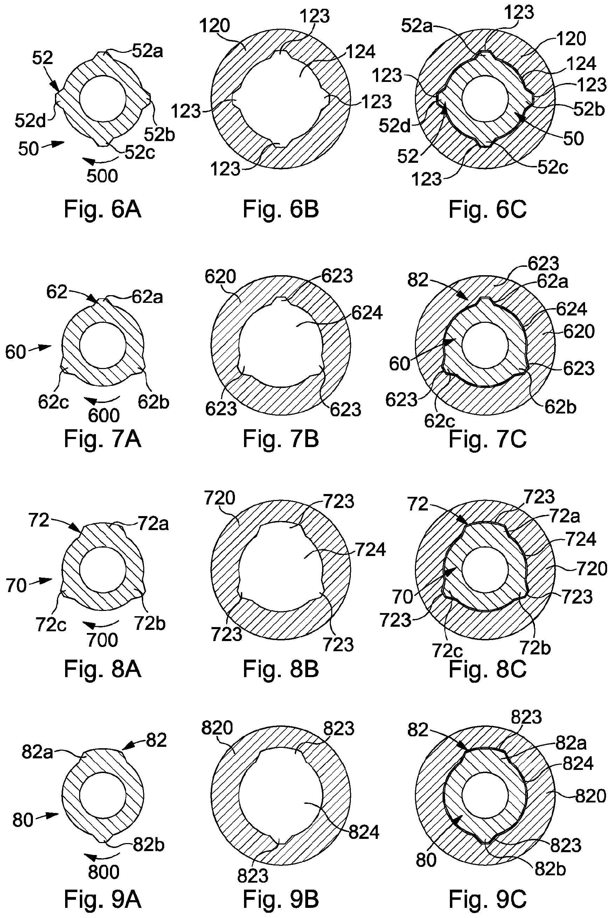

Figure 6A is a schematic view showing the circumferential distribution of the lugs 52 of the female luer lock fitting 50 of Figures 5A and 5B. The lugs 52 comprise a first lug 52a, a second lug 52b spaced 90deg from the first lug 52a in a clockwise direction 500, a third lug 52b spaced 90deg from the second lug 52b in the clockwise direction 500, and a fourth lug 52d spaced 90deg from the third lug 52c in the clockwise direction 500. The first lug 52a, second lug 52b, third lug 52c and fourth lug 52d are of equal size. Figure 6B is a top view of the through-hole 124 of the cap 120 of the one-piece fluid line connector 10 shown in Figures 1A to 2B. The through-hole 124 has four slots 123 configured to receive the four lugs 52a, 52b, 52c, 52d of the female luer lock fitting 50, the slots 123 being correspondingly positioned with equal spacing around the circumference of the through-hole 124. Figure 6C is a schematic view of the through-hole 124 with the female luer lock fitting 50 received therein. In use, the female luer lock fitting 50 may pass axially through the through-hole 124 to engage the male luer lock fitting 112, whereby rotation of the female luer lock fitting 50 is prevented whilst the lugs 52 are adjacent the slots 123 of the through-hole 124.

The circumferential distributions of the lugs 52 of the female luer lock fitting 50 and the corresponding slots 123 of the through-hole 124 are not limited to the configurations described above. Indeed, in alternative embodiments, other suitable configurations may be employed. For example, there may be fewer or more than four lugs 52 and the lugs 52 may be sized, shaped and/or circumferentially distributed differently around the female luer lock fitting 50.

Figures 7A to 9C show alternate configurations of the lugs and corresponding slots in accordance with alternative embodiments of the present invention.

In the embodiment shown in Figures 7A to 7C, lugs 62 comprise a first lug 62a, a second lug 62b spaced 120deg from the first lug 62a in a clockwise direction 600, and a third lug 62b spaced 120deg from the second lug 62b in the clockwise direction 600. A through-hole 624 has three slots 623 configured to receive the three lugs 62a, 62b, 62c of a female luer lock fitting 60, the slots 623 being correspondingly positioned with equal spacing around the circumference of the through-hole 624.

In the embodiment shown in Figures 8A to 8C, lugs 72 comprise a first lug 72a, a second lug 72b spaced 120deg from the first lug 72a in a clockwise direction 700, and a third lug 72b spaced 120deg from the second lug 72b in the clockwise direction 700. The first lug 72a is wider than the second lug 72b and third lug 72c. A through-hole 724 has three slots 723 configured to receive the three lugs 72a, 72b, 72c of a female luer lock fitting 70, the slots 723 being correspondingly positioned with equal spacing around the circumference of the through-hole 724.

In the embodiment shown in Figures 9A to 9C, lugs 82 comprise a first lug 82a and a second lug 82b spaced 180deg from the first lug 82a in a clockwise direction 800. The first lug 82a is wider than the second lug 82b. A through-hole 824 has two slots 823 configured to receive the two lugs 82a, 82b of a female luer lock fitting 80, the slots 823 being correspondingly positioned with equal spacing around the circumference of the through-hole 824.

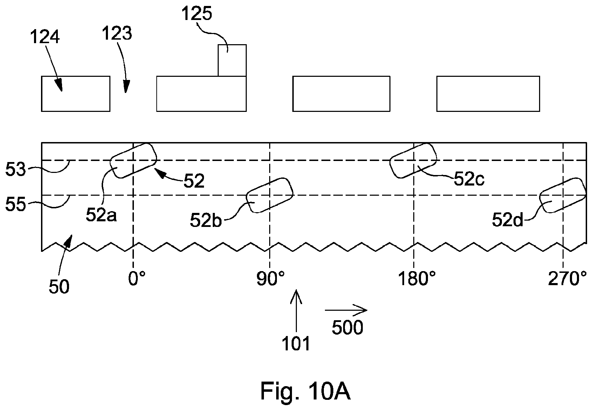

Figure 10A is a schematic overview of the radially external surface of the female luer lock fitting 50 of Figure 6A and the radially internal surface of the through-hole 124 of Figure 6B, where the female luer lock fitting 50 is in a pre-use configuration prior to engaging the one-piece fluid line connector 10. In Figure 10A, the circumferential surface area is flattened out to help show the axial and circumferential relationship of the lugs 52 of the female luer lock fitting 50. The absence of an internal surface area of the through-hole 124 is indicative of the slots 123 configured to receive the lugs 52.The first lug 52a is positioned in a first radial plane 53. The third lug 52c is positioned in the first radial plane 53 at a 180deg circumferential spacing from the first lug 52a. The second lug 52b is positioned in a second radial plane 55 at a 90deg circumferential spacing from the first lug 52a. The fourth lug 52d is positioned in the second radial plane 55 at a 270deg circumferential spacing from the first lug 52a. The first radial plane 53 is positioned axially forward of second radial plane 55. In use, the lugs 52 move forwardly in the axial direction 101 to pass through the slots 123 of the through-hole 124 and engage the thread 114 of the male luer lock fitting 112.

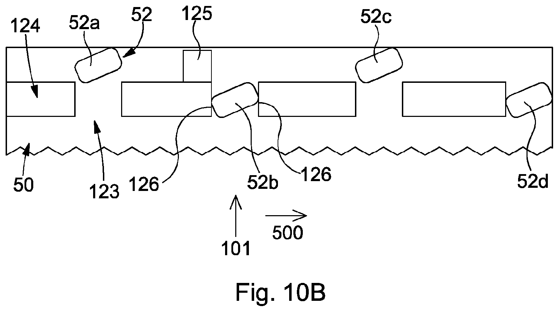

Figure 10B is a schematic overview of the arrangement shown in Figure 10A in a partially connected configuration, whereby the lugs 52 are partially received by the through-hole 124. In the configuration shown in Figure 10B, the first lug 52a and third lug 52c are axially forward of the through-hole 124, and the second lug 52b and fourth lug 52d are in axial alignment with the through-hole 124. In this configuration, the second lug 52b and fourth lug 52d are prevented from moving in the circumferential direction 500 by slot walls 126 of the through-hole 124, consequently the female luer lock fitting 50 is prevented from rotating relative to the cap 120 of the one-piece fluid line connector 10.

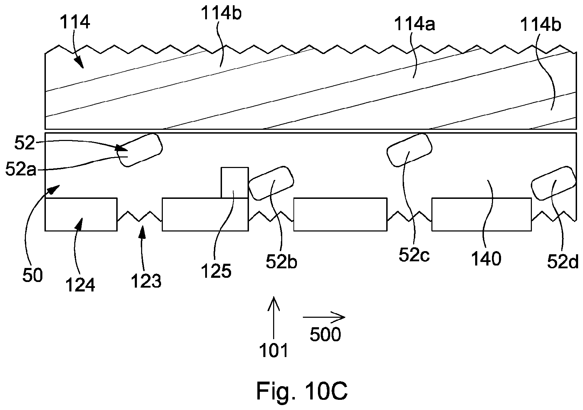

Figure 10C is a schematic overview of the arrangement shown in Figure 10A in a pre-threaded configuration and the radially internal surface of the thread 114 of the one-piece fluid line connector 10 in the aligned configuration, whereby the lugs 52 are received by a void 140 of the one-piece fluid line connector 10. The void 140 (also seen in Figure 2B) is positioned and extends axially between the through-hole 124 of the cap 120 and the thread 114 of the male luer lock fitting 112 of the main body 110. The void 140 is sized to receive the lugs 52 of the female luer lock fitting 50 and permit free rotation of the female luer lock fitting 50 such that the lugs 52 may engage the thread 114 of the male luer lock fitting 112. Preferably the void 140 has a depth of about 2.5mm. In use, the lugs 52 may move relative to the thread 114 in the circumferential direction 500 and axial direction 101 such that the thread 114 receives the lugs 52.

In the embodiment shown in the figures, the thread 114 is a double helix thread including a first helical thread 114a and a second helical thread 114b. The slots 123 of the through-hole 124 are positioned such that, when in the arrangement shown in Figure 10C, the lugs 52 are aligned with the thread 114. An advantage of such an alignment is that the lugs 52 do not necessarily require further rotation once they have passed through the through-hole 124 to subsequently align with the thread 114. This may represent a time saving for a user and reduce the risk of jamming. The first lug 52a and fourth lug 52d are helically aligned with the first helical thread 114a. The second lug 52b and third lug 52c are helically aligned with the second helical thread 114b. In a partially-threaded configuration (not shown), the first lug 52a is received by the first helical thread 114a, the third lug 52c is received by the second helical thread 114b, and the second lug 52b and fourth lug 52d remain received in the void 140. In a threaded configuration (not shown), the first lug 52a and fourth lug 52d are both received by the first helical thread 114a, and the second lug 52b and third lug 52c are both received by the second helical thread 114b.

The cap 120 further includes a stopper 125 positioned adjacent to one of the slots 123 (as seen in Figures 10A, 10B and 10C), the stopper 125 is configured to guide the lugs 52 through the through-hole 124 upon removal of the female luer lock fitting 50 from the one-piece fluid line connector 10. As shown in Figure 10C, the stopper 125 is positioned circumferentially rearward of the slot 123 corresponding to the second lug 52b and extends axially forward of the through hole 124 to an axial distance corresponding to the size of one lug 52. In certain embodiments, the stopper 125 may extend to an axial distance equal to the axial length of the void 140. The stopper 125 prevents the second lug 52b from rotating counter clockwise such that when the second lug 52b is adjacent to the stopper 125 (as shown in Figure 10C), the lugs 52 are in axial alignment with the slots 123 of the through-hole 124. An advantage of the stopper 125 is that the time taken to remove the lugs 52 from the void 140 via the through-hole 124 may be reduced as the user cannot over-rotate the lugs 52 beyond the stopper 125. Without the stopper 125, the user may rotate the lugs 52 within the void 140 to align the lugs 52 with the slots 123 of the through-hole 124, which may result in additional time being taken to remove the female luer lock fitting 50 from the one-piece fluid line connector 10. Further, by preventing over-rotation, the risk that lugs 52 may get jammed within the void 140 is significantly reduced.

The cap 120 is rotationally fixed relative to the main body 110 by the living hinge 130. This is advantageous in that as a user removes the lugs 52 of the female luer lock fitting 50 from the thread 114 of the male luer lock fitting 112, the slots 123 are aligned with the lugs 52 i.e. the cap 120 has not rotated to a misaligned position. This means that the user only needs to rotate the female luer lock fitting 50 to the point where the lugs 52 exit the thread 114, which is a time saving in not needing to further rotate the female luer lock fitting 50 to align the lugs 52 with the slots 123 of the through-hole 124. Once aligned with the slots 123 of the through-hole 124, the lugs 52 may pass axially through the through-hole 124 and allow the female luer lock fitting 50 to separate from the one-piece fluid line connector 10. By being rotationally fixed, there is minimal risk that the cap 120 may rotate with the female luer lock fitting 50 as the user rotates the female luer lock fitting 50. Advantageously, with the one-piece fluid connector 10 stationary, the female luer lock fitting 50 may be removed from the one-piece fluid line connector 10 using one hand.

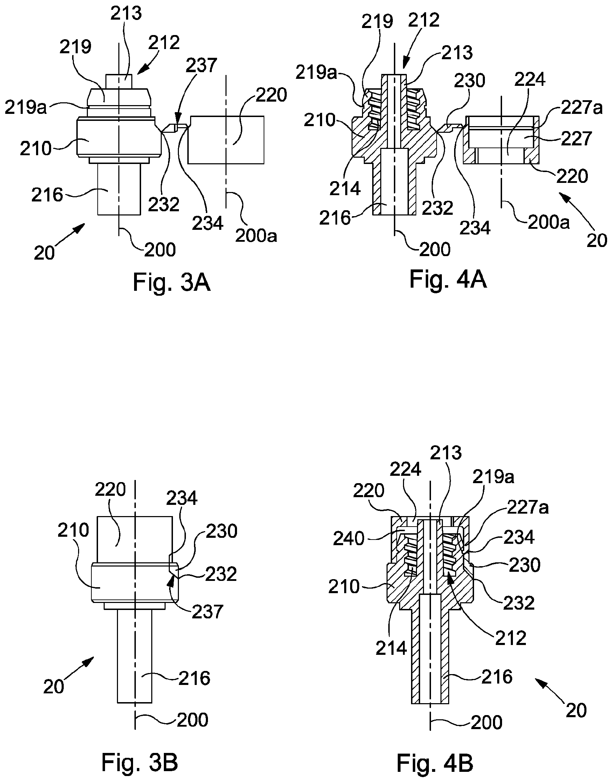

Figures 3A, 3B, 4A and 4B show a one-piece fluid line connector 20 according to another embodiment of the present invention. The one-piece fluid line connector 20 shares features with the one-piece fluid line connector 10 described above, and such shared features are numbered using the same last two digits and a differing preceding digit. Differing or additional features of the one-piece fluid line connector 20 are described below. In certain embodiments, any or all of the differing or additional features of the one-piece fluid line connector 20 may be incorporated into the one-piece fluid line connector 10 described above.

Like the one-piece fluid line connector 10 described above, the one-piece fluid line connector 20 includes a main body 210 having a central axis 200, and a cap 220 coupled to the main body 210 by a living hinge 230. Figure 3B shows the one-piece fluid line connector 10 of Figure 3A in an aligned configuration, in which the cap 220 is aligned with the central axis 200 of the main body 210.

Figure 4A is a cross-sectional view of the one-piece fluid line connector 20 of Figure 3A. Like the main body 110 described above, the main body 210 includes a male luer lock fitting 212 having a luer taper 213 and a thread 214, and a conduit 216 in fluid communication with the male luer lock fitting 212. The main body 210 further includes a receivable portion 219 having an annular recess 219a. The cap 220 includes a through-hole 224 at one end and a fastening portion 227 at an end opposite the through-hole 224, the fastening portion 227 includes a flange 227a. Figure 4B is a cross-sectional view of the one-piece fluid line connector 20 of Figure 3B. In the aligned configuration as shown in Figure 4B, the fastening portion 223 of the cap 220 receives the receivable portion 219 of the main body 210, and the annular recess 219a receives the flange 227a. In certain embodiments, the receivable portion 219 may include a flange and the fastening portion 227 may include a recess.

Like the living hinge 130 described above, the living hinge 230 is pivotable about both a first end 232 and a second end 234 such to move the cap 220 from the position shown in Figures 3A and 4A, to the aligned position as shown in Figures 3B and 4B. The living hinge 230 also has a contact surface area 237 configured to abut against the main body 210 when in the aligned configuration shown in Figures 3B and 4B. The contact surface area 237 is configured to guide the movement of the cap 220 to align the longitudinal axis 200a of the cap 220 with the central axis 200 of the main body 210.

Like the male luer lock fitting 112 described above, the luer taper 213 of the male luer lock fitting 212 is configured to be received by the receiving portion 54 of the female luer lock fitting 50, the thread 214 of the male luer lock fitting 212 is configured to receive the lugs 52 of the female luer lock fitting 50, and the conduit 216 of the main body 212 is configured for attaching the main body 210 to a fluid feed container (not shown). Consequently, in use, the main body 210 permits fluid flow between a fluid feed container (not shown) and a fluid feed line (not shown) via the female luer lock fitting 50.

The receivable portion 219 of the main body is configured such that, in the aligned configuration, the receivable portion 219 is radially internal of the cap 220 as shown in Figure 4B. In the aligned configuration as shown in Figure 4B, the recess 219a may engage with the flange 227a of the cap 220 such to form a snap-fit fastening between the cap 220 and the receivable portion 219 of the main body 210. The snap-fit fastening ensures that the cap 220 remains received by the receivable portion 219 during normal use of the one-piece fluid line connector 20. In certain embodiments, it is intended that the cap 220 will not be removed from the main body 210 once it is fastened to the main body 210.

Like the through-hole 124 of the cap 110 described above, the through-hole 224 of the cap 220 is configured such that, in the aligned configuration, the shape of the through-hole 224 permits a correspondingly-shaped female luer lock fitting (not shown) to pass therethrough and engage the male luer lock fitting 212 of the main body 210. Conversely, a non-correspondingly-shaped female luer lock fitting will not be capable of passing through the through-hole 124.

Like the one-piece fluid line connector 10 of the above described embodiment, the one-piece fluid line connector 20 includes, in the aligned configuration as shown in Figure 4B, a void 240 positioned between the through-hole 224 and the thread 214. The void 240 is sized to receive the lugs 52 of the female luer lock fitting 50 and permit free rotation of the female luer lock fitting 50. Preferably the void 240 has an axial depth of about 2.5mm

The through-hole 224, void 240 and thread 214 of the one-piece fluid line connector 20 interact with the female luer lock fitting 50 in a manner as described above with relation to the through-hole 124, void 140 and thread 114 of Figures 10A, 10B and 10C.

In certain embodiments, the thread of the male luer lock fitting may be a single helix, a triple helix or any other suitable threaded pattern.

In certain embodiments, the male luer lock fitting may not have a thread, and instead have alternative means of securing the female luer lock fitting to the male luer lock fitting in use. For example, the male luer lock fitting may form a bayonet-type connection with the female luer lock fitting.

In certain embodiments, the through-hole of the cap may be threaded and aligned with the thread of the male luer lock fitting. In such embodiments, the female luer lock fitting may be rotated through the through-hole and into the thread of the male luer lock fitting.

In certain embodiments, the through-hole may be positioned adjacent to the beginning of the thread of the male luer lock fitting, such that there is no void between the through-hole and the thread.

In certain embodiments, the through-hole of the cap may include more than one stopper or no stopper at all.

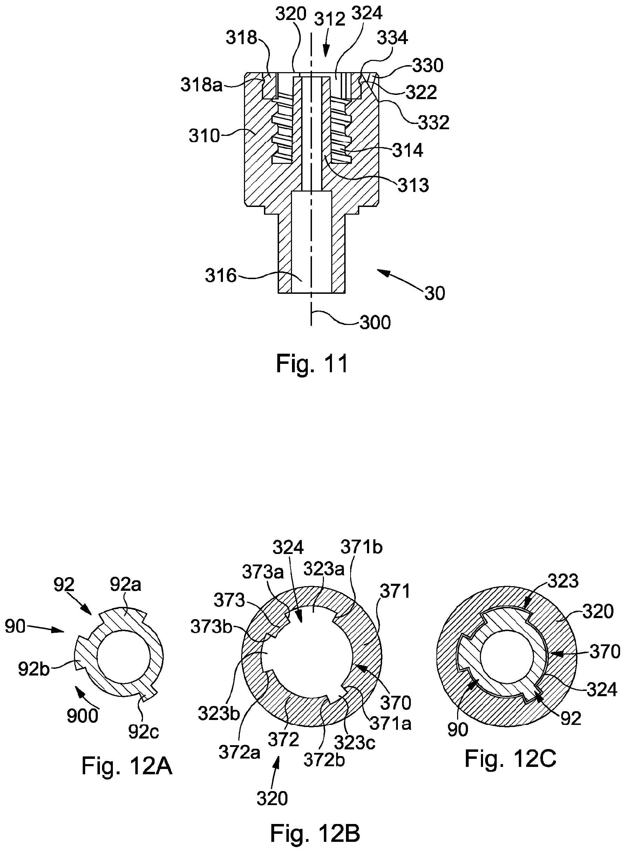

Figure 11 shows a cross-sectional view of a one-piece fluid line connector 30 according to another embodiment of the present invention. The one-piece fluid line connector 30 shares features with the one-piece fluid line connector 10 described above and shown in Figures 1A to 2B, and such shared features are numbered using the same last two digits and a differing preceding digit. Differing or additional features of the one-piece fluid line connector 30 are described below. In certain embodiments, any or all of the differing or additional features of the one-piece fluid line connector 30 may be incorporated into the one-piece fluid line connector 10 described above.

The one-piece fluid connector 30 includes a cap 320 coupled to a main body 310 of the one-piece fluid line connector 30 by a living hinge 330. Like the one-piece fluid line connector 10 of Figures 1B and 2B, the one-piece fluid line connector 30 is shown in an aligned configuration in which the cap 320 is aligned with a central axis 300 of the main body 310. The cap 320 includes a through-hole 324 configured so that a correspondingly-shaped female luer lock fitting may pass therethrough to engage the main body 310 of the one-piece fluid line connector 30. In the aligned configuration shown in Figure 11, the cap 320 is positioned adjacent to the beginning of a thread 314 of the male luer lock fitting 312. As such, the axial spacing between the cap 320 and the thread 314 is less than the axial height of a lug of a correspondingly-shaped female luer lock fitting to substantially prevent a lug from rotating in a space between the cap 320 and the thread 314. An advantage of having the cap 320 positioned adjacent the thread 314 is that the risk that lugs may get jammed within the one-piece fluid line connector 30 is substantially eliminated as there is no space between the cap 320 and the thread 314 for jamming to occur. This may advantageously reduce the likelihood of failure of the one-piece fluid line connector 30 when being used to connect to a female luer lock fitting. Further, eliminating the risk of the lugs jamming within the one-piece fluid line connector 30 may represent a time saving for a user of the one-piece fluid line connector.

Figure 12A is a schematic view showing the circumferential distribution of lugs 92 around an example female luer lock fitting 90 suitable for connecting to the one-piece fluid line connector 30 of Figure 11. The female luer lock fitting 90 shares features with the female luer lock fitting 50 described above and shown in Figures 5A and 5B, and such shared features are numbered using the same second digit and a differing first digit. Differing or additional features of the female luer lock fitting 90 are described below. The lugs 92 comprise a first lug 92a having an angular extent of 60 degrees around a circumference of a female luer lock fitting 90, a second lug 92b having an angular extent of 40 degrees, and a third lug 92c having an angular extent of 20 degrees.

Figure 12B is a top view of the through-hole 324 of the cap 320 of the one-piece fluid line connector 30 shown in Figure 11. The through-hole 324 has slots 323 configured to receive the lugs 92 of the female luer lock fitting 90, the slots 323 being correspondingly positioned around the circumference of the through-hole 324 such that a first slot 323a is configured to receive the first lug 92a, a second slot 323b is configured to receive the second lug 92b and a third slot 323c is configured to receive the third lug 92c. As such, the angular extent of the first slot 323a is 60 degrees, the angular extent of the second slot 323b is 40 degrees, and the angular extent of the third slot 323c is 20 degrees.

The distance from the axial centre of the through-hole to the perimeter of the through-hole is variable about a perimeter of the through-hole. Consequently, the through-hole has one or more adjacent sectors with different radii, wherein the distance from the axial centre of the through-hole to the outermost point of one sector is different to that of an adjacent sector. The slots 323 correspond with one or more of the adjacent sectors of the through-hole 324 that allow a correspondingly-shaped lug of a female luer lock fitting to pass therethrough.

Figure 12C is a schematic view of the through-hole 324 with the female luer lock fitting 90 received therein. In use, the female luer lock fitting 90 may pass through the through-hole 324 to engage the male luer lock fitting 312.

The angular extent of the first lug 92a may provide structural support for the female luer lock fitting 90 when received by the one-piece fluid line connector 30, therefore, advantageously, the shorter lugs 92b, 92c may be circumferentially distributed in other examples so as to create unique variations of the female luer lock fitting 90. Indeed, a different circumferential distribution may require a different axial distribution so that all lugs may be helically aligned with the thread of the one-piece fluid line connector.

Having three lugs of different angular extents permits a large number of possible unique variations of the female luer lock fitting. This may advantageously increase the availability of unique fluid connections to a user so to reduce the likelihood of undesired fluid connections being made.

In other examples, the angular extent of each lug may be different to one another in any combination so long as the female luer lock fitting may connect with the one-piece fluid line connector. In further examples, the third lug may have the same angular extent as the second lug.

In certain embodiments, the female luer lock fitting may include a visual or tactile indicator (not shown) to aid a user in identifying the largest lug such to enable the user to align the largest lug with the largest slot when connecting the female luer lock fitting with the one-piece fluid line connector. In certain embodiments, the cap of the one-piece fluid line connector may include a visual or tactile indicator (not shown) to aid a user in identifying the correct orientation required of the female luer lock fitting such that the female luer lock fitting may pass therethrough. The visual or tactile indicator may comprise, for example, a rib or spline.

Alignment of a tactile indicator of the cap with a tactile indicator of the female luer lock fitting may permit the female luer lock fitting to pass through the through-hole of the one-piece fluid line connector. Consequently, in use, an operator may advantageously quickly and simply identify the orientation required so that the female luer lock fitting may be connected with the one-piece fluid line connector in less time than certain prior art fluid connections. This may reduce the difficulty and frustration associated with aligning certain prior art fluid line connectors. Further, it is advantageous to have a quick and simple method of connecting fluid lines as a single patient may require many (e.g. dozens) fluid line connections which need to be connected efficiently.

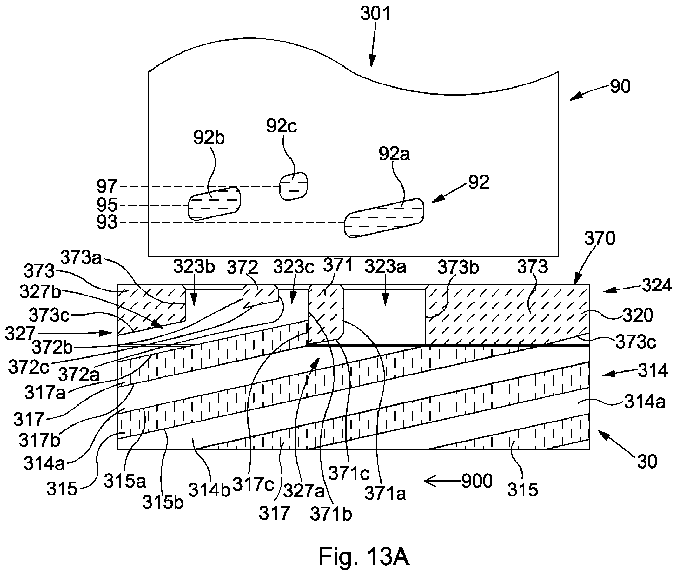

Figure 13A is a schematic overview of the radially external surface of the female luer lock fitting 90 of Figure 12A, the radially internal surface of the through-hole 324 of Figure 12B, and the thread 314 of the one-piece fluid line connector 30 of Figure 11. In Figure 13A, the female luer lock fitting 90 is in a pre-use configuration prior to engaging the one-piece fluid line connector 30. The circumferential surface area of the female luer lock fitting 90 is flattened out to help show the axial and circumferential relationship of the lugs 92. The absence of an internal surface area of the through-hole 324 is indicative of the slots 323 configured to receive the lugs 92. The first lug 92a is positioned in a first radial plane 93. The second lug 92b is positioned in a second radial plane 95 which is axially rearward of the first radial plane 93. The third lug 92c is positioned in a third radial plane 97 which is axially rearward of the second radial plane 95. In use, the lugs move in an axially forward direction 301 to enter the slots 323 of the through-hole 324. In other embodiments, two of the lugs may be in a first radial plane and the third lug may be in a second radial plane axially spaced from the first radial plane.

The slots 323 of the through-hole 324 are determined by radially inwardly extending portions 370 of the cap 320. The radially inwardly extending portions 370 comprise a first radial portion 371 having a first sidewall 371a and a second sidewall 371b, a second radial portion 372 having a first sidewall 372a and a second sidewall 372b, and a third radial portion 373 having a first sidewall 373a and a second sidewall 373b. The first slot 323a is positioned between the first sidewall 371a of the first radial portion 371 and the second sidewall 373b of the third radial portion 373. The second slot 323b is positioned between the first sidewall 373a of the third radial portion 373 and the second sidewall 372b of the second radial portion 372. The third slot 323c is positioned between the first sidewall 372a of the second radial portion 372 and the second sidewall 371b of the first radial portion 371.

The first sidewalls 371a, 372a, 373a are configured to prevent rotation of the female luer lock fitting 90 in a clockwise direction 900 when any of the lugs 92 are axially adjacent one of the first sidewalls 371a, 372a, 373a. The second sidewalls 371b, 372b, 373b are configured to prevent rotation of the female luer lock fitting 90 in a direction opposite the clockwise direction 900 when any of the lugs 92 are axially adjacent any of the second sidewalls 371b, 372b, 373b.

In use, when any of the lugs 92 is positioned in the corresponding slot 323a, 323b, 323c, the female luer lock fitting 90 is substantially prevented from rotating relative to the cap 320.

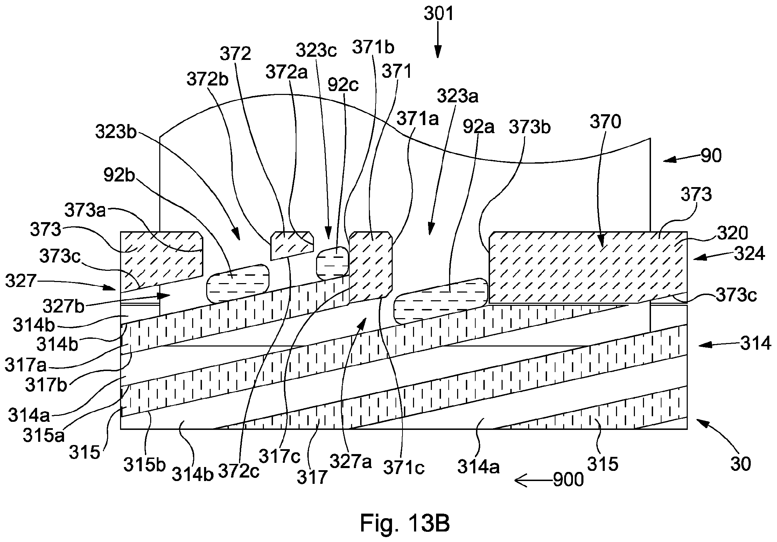

Figure 13B is a schematic overview of the arrangement shown in Figure 13A in an intermediate configuration, wherein the female luer lock fitting 90 may be rotated in the clockwise direction 900 to engage the lugs 92 with the thread 314 of the male luer lock fitting 312, or the female luer lock fitting 90 may be moved axially in a direction opposite the forward direction 301 to remove the female luer lock fitting 90 from the one-piece fluid line connector 30. In the configuration shown in Figure 13B, the first lug 92a is restrained from rotating in the counterclockwise direction by the second sidewall 373b of the third radial portion 373 and the third lug 92c is restrained from rotating in the counterclockwise direction by the second sidewall 372b of the second radial portion 372. An advantage of the second sidewall 371b, 372b, 373b is that it is not possible for a user to over-rotate the lugs 92 when removing the lugs 92 from the thread 314. Such an advantage may reduce the time taken to disconnect the female luer lock fitting 90 from the one-piece fluid line connector 30.

The cap 320 has a guide portion 327 configured to determine helical movement of the lugs 92 between the cap 320 and the thread 314 when the female luer lock fitting 90 is within the cap 320 and the lugs 92 are helically aligned with the thread 314. The guide portion 327 may cooperate with the thread 314 to effectively form a continuation of the thread 314 within the cap 320. To achieve this continuation of the thread 314, the cap 320 may be partially threaded and/or the cap 320 may receive a portion of the thread 314.

The thread 314 is a double helix thread comprising a first helical ridge 315 and a second helical ridge 317 spaced from the first helical ridge 315. The first helical ridge 315 comprises a first side 315a and a second side 315b opposite the first side 315a. The second helical ridge 317 comprises a first side 317a and a second side 317b opposite the first side 317a. A first helical thread 314a for receiving a lug is defined between the first side 315a of the first helical ridge 315 and the second side 317b of the second helical ridge 317. A second helical thread 314b is defined between the second side 315b of the first helical ridge 315 and the first side 317a of the second helical ridge 317.

In Figure 13B, the first lug 92a is helically aligned with the first helical thread 314a, and the second lug 92b and third lug 92c are each helically aligned with the second thread 314b. An advantage of such an alignment is that the female luer lock fitting 90 does not require further circumferential rotation once the first lug 92a has passed axially through the through-hole 324 to subsequently align with the thread 314. This may represent a time saving for a user when connecting or disconnecting the female luer lock fitting 90 with the one-piece fluid line connector 30.

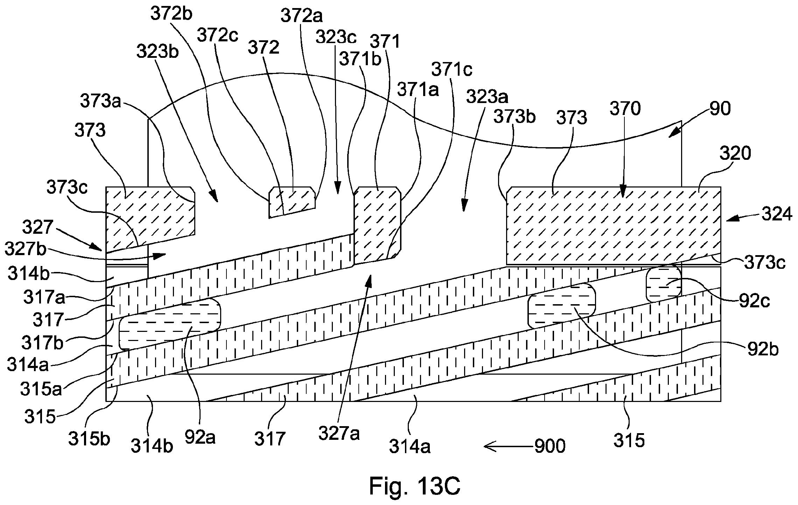

Fig 13C is a schematic overview of the arrangement shown in Figure 13A in a threaded configuration, wherein all of the lugs 92 are received by the thread 314 of the one-piece fluid line connector 30.

As shown in Figures 13A to 13C, the first radial portion 371 includes, at an end adjacent the thread 314 when in the aligned configuration, a sloped surface 371c having a sloped portion aligned with the second side 317b of the second ridge 317. In use, the sloped surface 371c of the first radial portion 371 and the first side 315a of the first helical ridge 315 of the thread 314 cooperate to define a first helical channel 327a to determine helical movement of the first lug 92a between the cap 320 and the first helical thread 314a as the female luer lock fitting 90 is rotated when the first lug 92a is helically aligned with the first helical thread 314a.

Each of the second radial portion 372 and the third radial portion 373 includes, at an end adjacent the thread 314 when in the aligned configuration, a sloped surface 372c, 373c aligned with the second side 315b of the second helical ridge 315.

The second helical ridge 317 is configured to be partially received by the cap 320 when the cap 320 is in the aligned configuration. The second helical ridge 317 is received by the cap 320 so that the second sidewall 371b of the first radial portion 371 substantially abuts an end 317c of the second helical ridge 317. In use, the sloped surface 373c of the third radial portion 373 and the first side 317a of the second helical ridge 317 cooperate to define a second helical channel 327b to determine helical movement of the second lug 92b and the third lug 92c between the cap 320 and the second helical thread 314b as the female luer lock fitting 90 is rotated when the second lug 92b and the third lug 92c are helically aligned with the second helical thread 314b. In use, the sloped surface 372c of the second radial portion 372 and the first side 317a of the second helical ridge 317 cooperate to determine helical movement of the third lug 92c between the cap 320 and the second helical thread 314b as the female luer lock fitting 90 is rotated when the third lug 92c is helically aligned with the second helical thread 314b.

The sloped surfaces 371c, 372c, 373c define a portion of the lowermost surface of the cap 320. That is to say, when the cap 320 is viewed along its axis from one end, the sloped surfaces 371c, 372c, 373c would be visible. In use, the lowermost surface of the cap 320 is configured to face the thread 314 when the cap 320 is in the aligned position.

The guide portion 327 of the cap 320 is defined by the sloped surfaces 371c, 372c, 373c of the radial portions 371, 372, 373. In other embodiments, the guide portion may be separate to the radial portions. For example, the guide portion may be axially separated from the radial portions.

The one-piece fluid line connector 30 is configured so that, in use, the female luer lock fitting 90 may move axially through the through-hole 324 so that the first lug 92a engages the first side 315a of the first helical ridge 315, and the second lug 92b and the third lug 92c engage the first side 317a of the second helical ridge 317. When the lugs 92 are engaged with their respective helical ridges 315, 317, and helically aligned with their respective helical thread 314a, 314b, the one-piece fluid line connector 30 may determine helical movement of the lugs 92 upon rotation of the female luer lock fitting 90 so that the lugs 90 move between the cap 320 and their respective helical threads 314a, 314b. During such helical movement, all of the lugs 92 are axially restrained by either or both of the sloped surfaces 371c, 372c, 373c and the thread 314 in at least one rotational position of the female luer lock fitting 90 within the cap 320.

By determining helical movement of the lugs between the thread and the cap, the likelihood of a lug not engaging a thread, or a lug being jammed within the cap, is substantially eliminated. Also, such a configuration aids the user in quickly and simply connecting or disconnecting the lugs and the thread. This is advantageous, particularly when a user of the one-piece fluid line connector is likely under pressure and/or multitasking (e.g. attempting to connect multiple fluid lines in a short time period), to reduce the difficulty and time taken involved to fluidly connect the female luer lock fitting with the one-piece fluid line connector. This may reduce the likelihood that an undesired or incomplete fluid line connection is made.

In other embodiments, the through-hole of the cap may comprise a cap thread where the cap thread forms the guide portion. One or more of the lugs may first move axially through the through-hole of the cap until they are helically aligned with the cap thread. When the one or more lugs engage the cap thread, the cap thread determines helical movement of the lugs into the thread of the main body as the female luer lock fitting is rotated. In such an embodiment, a portion of the cap thread defines a portion of the lowermost surface of the cap. The portion of the cap thread is configured to cooperate with the thread of the male luer lock fitting to determine helical movement between the cap and the thread.

In other embodiments, either or both of the helical ridges may be received by the cap when the cap is in the aligned configuration so long as the one-piece fluid line connector is capable of determining helical movement of the lugs when the female luer lock fitting is rotated and the lugs are helically aligned with the thread of the one-piece fluid line connector.

Indeed, in other embodiments, any of the sloped surfaces 371c, 372c, 373c may be one or more of straight, sloped and curved so long as the lugs 92 may move past the radial portions 370 when the female luer lock fitting 90 is helically rotated into the thread 314.

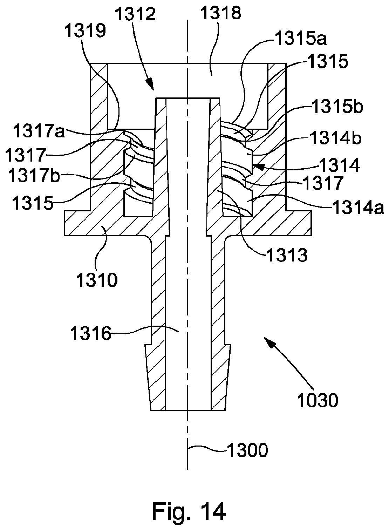

Figure 14 shows a cross-sectional view of a one-piece fluid line connector 1030 according to another embodiment of the present invention. The one-piece fluid line connector 1030 shares features with the one-piece fluid line connector 30 described above and shown in Figures 11 to 13C, and such shared features are numbered using the same three digits with an additional preceding digit. Differing or additional features of the one-piece fluid line connector 1030 are described below. In certain embodiments, any or all of the differing or additional features of the one-piece fluid line connector 1030 may be incorporated into the one-piece fluid line connector 30.

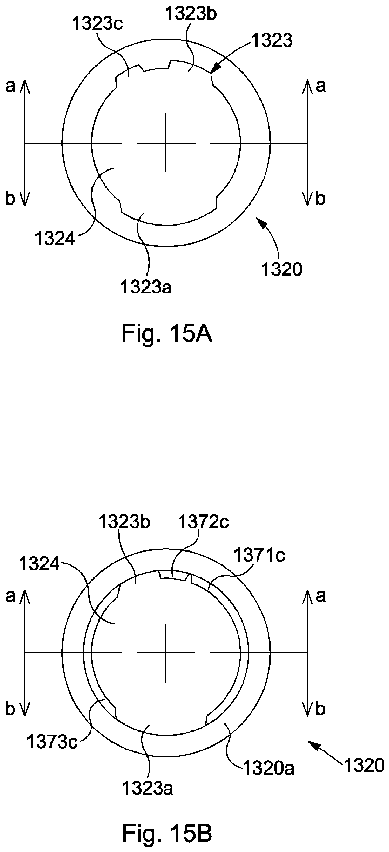

The one-piece fluid line connector 1030 includes a main body 1310. The main body 1310 includes a male luer lock fitting 1312 having a luer taper 1313 and a thread 1314, a conduit 1316 in fluid communication with the male luer lock fitting 1312, and a cap receiving portion 1318. The cap receiving portion 1318 includes an abutment surface 1319 configured so that, when a cap 1320 (see Figures 15A to 15D) of the one-piece fluid line connector 1030 is in an aligned position, the cap 1320 is substantially adjacent to the abutment surface 1319.

Like the thread 314 of Figures 11 and 13A to 13C, the thread 1314 is a double helix thread comprising a first helical ridge 1315 and a second helical ridge 1317 spaced from the first helical ridge 1315. In the embodiment shown in Figure 14, the first helical ridge 1315 partially extends into the cap receiving portion 1318. The second helical ridge 1317 partially extends into the cap receiving portion 1318 of the one-piece fluid line connector 1030, although this is not shown in Figure 14. The first helical ridge 1315 comprises a first side 1315a and a second side 1315b opposite the first side 1315a. The second helical ridge 1317 comprises a first side 1317a and a second side 1315b opposite the first side 1317a. A first helical thread 1314a for receiving a lug is defined between the first side 1315a of the first helical ridge 1315 and the second side 1317b of the second helical ridge 1317. A second helical thread 1314b is defined between the second side 1315b of the first helical ridge 1315 and the first side 1317a of the second helical ridge 1317.

Figure 15A shows a schematic top view of the cap 1320 of the one-piece fluid line connector 1030 of Figure 14. Like the cap 320 of Figure 12B, the cap 1320 includes a through-hole 1324 configured so that a correspondingly-shaped female luer lock fitting may pass therethrough to engage the main body 1310 of the one-piece fluid line connector 1030. The through-hole 1324 has slots 1323 configured to receive lugs of a correspondingly-shaped female luer lock fitting. Like the slots 323 of through-hole 324, the slots 1323 comprise a first slot 1323a, a second slot 1323b and a third slot 1323c. Indeed, in other embodiments, the slots 1323 may be differently circumferentially spaced, and/or have a differing radial extent about the circumference of the through-hole 1324.

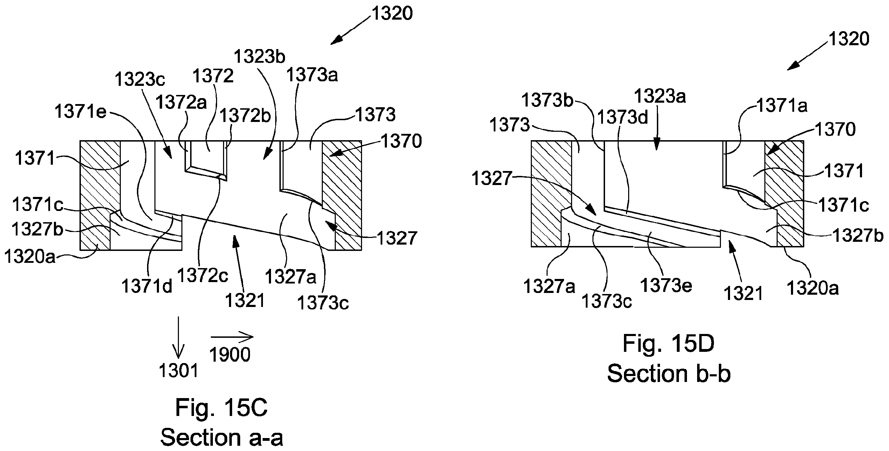

Figure 15C is a cross-sectional view along line a-a of Figure 15A. Figure 15D is a cross-sectional view along line b-b of Figure 15A. Like the cap 320 of the previous embodiment, the cap 1320 includes radially inwardly extending portions 1370 that determine the slots 1323 of the through-hole 1324. The cap 1320 includes an abutment surface 1320a configured to substantially abut the abutment surface 1319 of the cap receiving portion 1318 of the one-piece fluid line connector 1030, when the cap 1320 is in the aligned position.

The radially inwardly extending portions 1370 comprise a first radial portion 1371 having a first sidewall 1371a and a second sidewall 1371b, a second radial portion 1372 having a first sidewall 1372a and a second sidewall 1372b, and a third radial portion 1373 having a first sidewall 1373a and a second sidewall 1373b. The first slot 1323a is positioned between the first sidewall 1371a of the first radial portion 1371 and the second sidewall 1373b of the third radial portion 1373. The second slot 1323b is positioned between the first sidewall 1373a of the third radial portion 1373 and the second sidewall 1372b of the second radial portion 1372. The third slot 1323c is positioned between the first sidewall 1372a of the second radial portion 1372 and the second sidewall 1371b of the first radial portion 1371.

The first sidewalls 1371a, 1372a, 1373a are configured to prevent rotation of a female luer lock fitting in a clockwise direction 1900 when any lugs of the female luer lock fitting are axially adjacent one of the first sidewalls 1371a, 1372a, 1373a. The second sidewalls 1371b, 1372b, 1373b are configured to prevent rotation of the female luer lock fitting in a direction opposite the clockwise direction 1900 when any lugs of the female luer lock fitting are axially adjacent any of the second sidewalls 1371b, 1372b, 1373b.

The cap 1320 includes a thread receiving portion 1321 configured to receive a portion of the thread 1314 when the cap 1320 is in the aligned position. The portion of the thread receiving portion 1321 shown in Figure 15C is configured to receive a portion of the first helical ridge 1315 of the thread 1314. The portion of the thread receiving portion 1321 shown in Figure 15D is configured to receive a portion of the second helical ridge 1317 of the thread 1314.

Like the cap 320 of the previous embodiment, the cap 1320 has a guide portion 1327 configured to determine helical movement of lugs between the cap 1320 and the thread 1314 when a female luer lock fitting is within the cap 1320 and the lugs are helically aligned with the thread 1314. The guide portion 1327 is configured to cooperate with the thread 1314 to effectively form a continuation of the thread 1314 between the thread 1314 and the cap 1320.

As shown in Figures 15C and 15D, the first radial portion 1371 includes a first sloped surface 1371c configured to be helically aligned with the second side 1315b of the first helical ridge 1315 when the cap 1320 is in the aligned position. When the cap 1320 is in the aligned position, the first sloped surface 1371c and the first side 1317a of the second helical ridge 1317 cooperate to define a second helical channel 1327b. The second helical channel 1327b is effectively a continuation of the second helical thread 1314b.

As shown in Figure 15C, the second radial portion 1372 includes a first sloped surface 1372c configured to helically align with the second side 1317b of the second helical ridge 1317 when the cap 1320 is in the aligned position. As shown in Figure 15C and Figure 15D, the third radial portion 1373 includes a first sloped surface 1373c configured to be helically aligned with the second side 1315b of the first helical ridge 1315 when the cap 1320 is in the aligned position. When the cap 1320 is in the aligned position, the first sloped surface 1372c of the second radial portion 1372, the first sloped surface 1373c of the third radial portion 1373, and the first side 1315a of the first helical ridge 1315 cooperate to define a first helical channel 1327a. The first helical channel 1327a is effectively a continuation of the first helical thread 1314a.

Figure 15B shows a bottom view of the cap 1320. The first sloped surfaces 1371c, 1372c, 1373c and the abutment surface 1320a define the lowermost surface of the cap 1320. The lowermost surface of the cap 1320 is configured to face the thread 1314 when the cap 1320 is in the aligned position.

As shown in Figure 15C, the first radial portion 1371 includes a second sloped surface 1371d configured to helically align with the second side 1315b of the first helical ridge 1315 when the cap 1320 is in the aligned position. The first sloped surface 1371c and the second sloped surface 1371d cooperate to provide a first cap ridge 1371e within the cap 1320. When the cap 1320 is in the aligned position, the first cap ridge 1371e is effectively a continuation of the first helical ridge 1315. As such, the first cap ridge 1371e is configured to substantially abut the first helical ridge 1315 when the cap is in the aligned position. In use, as a lug moves axially through the third slot 1323c in the direction 1301, the lug may be axially restrained by the first cap ridge 1371e.

As shown in Figure 15D, the third radial portion 1373 includes a second sloped surface 1371d configured to helically align with the second side 1317b of the second helical ridge 1317 when the cap 1320 is in the aligned position. The first sloped surface 1373c and the second sloped surface 1373d cooperate to provide a second cap ridge 1373e within the cap 1320. When the cap 1320 is in the aligned position, the second cap ridge 1373e is effectively a continuation of the second helical ridge 1317. As such, the second cap ridge 1373e is configured to substantially abut the second helical ridge 1317 when the cap is in the aligned position. In use, as a lug moves axially through the first slot 1323a in the direction 1301, the lug may be axially restrained by the second cap ridge 1373e.

The second sloped surfaces 1371d, 1373d define a portion of the uppermost surface of the cap 1320. The uppermost surface of the cap 1320 is configured to face away from the thread 1314 when the cap 1320 is in the aligned position.

The guide portion 1327 is defined by the first sloped surfaces 1371c, 1372c, 1373c and the second sloped surfaces 1371d, 1373d.

In the embodiment shown in Figures 15C and 15D, the first cap ridge 1371e and the second cap ridge 1373e do not undercut any of the radial portions 1370. That is to say, each radial portion 1371, 1372, 1373 is circumferentially spaced from another across the axial length of the cap 1320. This cap configuration provides a relatively simple shape to manufacture, which may advantageously reduce the time and cost of manufacture. For high volume manufacture, a small time or cost saving per unit may be highly advantageous.

In other embodiments, all of the radial portions may comprise a cap ridge. In some embodiments, one or more cap ridges may undercut one or more radial portions and in such an embodiment, it may not be necessary for the cap to receive the thread.

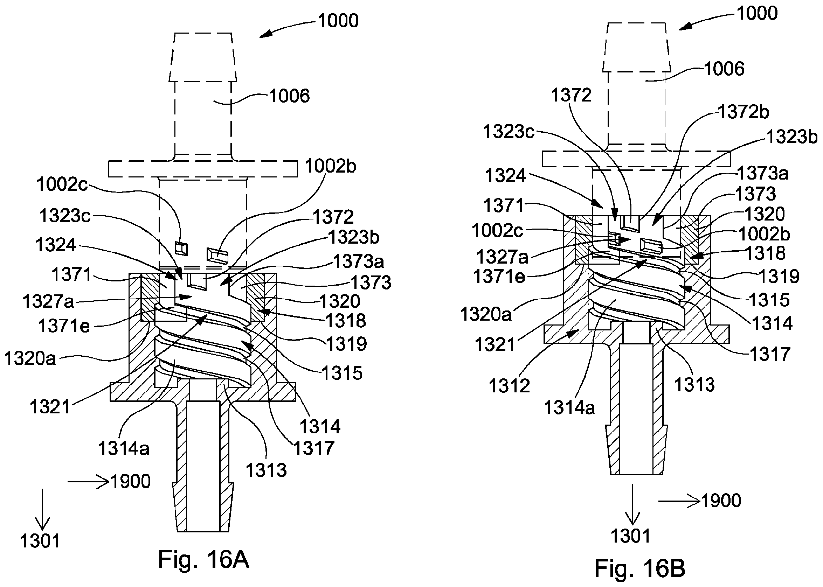

Figure 16A is a schematic cross-sectional overview showing the relationship between the one-piece fluid line connector 1030 and an example female luer lock fitting 1000. Figure 16A shows the one-piece fluid line connector 1030 in the aligned position wherein the cap 1320 is aligned with the central axis 1300 of the main body 1310 and adjacent the thread 1314. The cap 1320 is received by the cap receiving portion 1318 so that the abutment surface 1319 of the cap receiving portion 1318 abuts the abutment surface 1320a of the cap 1320. In the aligned position, the first helical ridge 1315 and the second helical ridge 1317 are each received by the thread receiving portion 1321 of the cap 1320. As shown in Figure 16A, the first cap ridge 1371e effectively forms a continuation of the first helical ridge 1315.

In Figure 16A, the luer taper 1313 is only partially shown in order to enable the relationship between the thread 1314, the cap 1320 and the female luer lock fitting 1000 to be clearly understood.

The female luer lock fitting 1000 shares features with the female luer lock fitting 50 and 90 described above and shown in Figures 5A, 5B and 13A to 13C respectively. Such shared features are numbered using the same final digit with differing three preceding digits. Differing or additional features of the female luer lock fitting 1000 are described below. In certain embodiments, any or all of the differing or additional features of the female luer lock fitting 1000 may be incorporated into the female luer lock fitting 1000. Like the female luer lock fitting 50 or 90, the female luer lock fitting 1000 includes a first lug 1002a (not shown), a second lug 1002b and a third lug 1002c. The body of the female luer lock fitting 1000 is shown in dotted lines, whilst the second lug 1002b and the third lug 1002c are shown in solid lines.

In Figure 16A, the female luer lock fitting 1000 is in a pre-use configuration prior to engaging the one-piece fluid line connector 1030. The first lug 1002a (not shown) is axially aligned with the first slot 1323a (not shown), the second lug 1002b is axially aligned with the second slot 1323b and the third lug 1002c is axially aligned with the third slot 1323c. In use, the female luer lock fitting 1000 may move axially in the direction 1301 to enter the through-hole 1324 of the one-piece fluid line connector 1030. When any of the lugs 1002 is positioned in the corresponding slot 1323a, 1323b, 1323c the female luer lock fitting 1000 may be substantially prevented from rotating relative to the cap 1320. For example, in an intermediate position (not shown), the second lug 1002b may be adjacent both the second sidewall 1372b of the second radial portion 1372 and the first sidewall 1373a of the third radial portion 1373. In such a position, the second lug 1002 may be circumferentially constrained by the respective radial portions 1372, 1373, and, consequently, the female luer lock fitting 1000 is also circumferentially constrained relative to the cap 1320.

Figure 16B is a schematic overview of the arrangement shown in Figure 16A in a pre-threaded configuration, wherein the female luer lock fitting 1000 may be rotated in the clockwise direction 1900 to engage the lugs 1002 with the thread 1314 of the male luer lock fitting 1312, or the female luer lock fitting 1000 may be moved axially in a direction opposite the direction 1301 to remove the female luer lock fitting 1000 from the one-piece fluid line connector 1030.

In Figure 16B, the second lug 1002b and the third lug 1002c are each helically aligned with the first helical channel 1327a and the first helical thread 1314a. In the position shown in Figure 16B, the third lug 1002c may be axially constrained by the first cap ridge 1371e of the first radial portion 1371, and the second lug 1002b may be axially constrained by the first helical ridge 1315 of the thread. The first lug 1002a (not shown) may be axially constrained by the second cap ridge 1373e of the third radial portion 1373.

In at least one rotational position of the female luer lock fitting 1000 within the one-piece fluid line connector 1030, the radial portions 1370 cooperate with the thread 1314 to axially constrain at least one of the lugs 1002a, 1002b, 1002c.

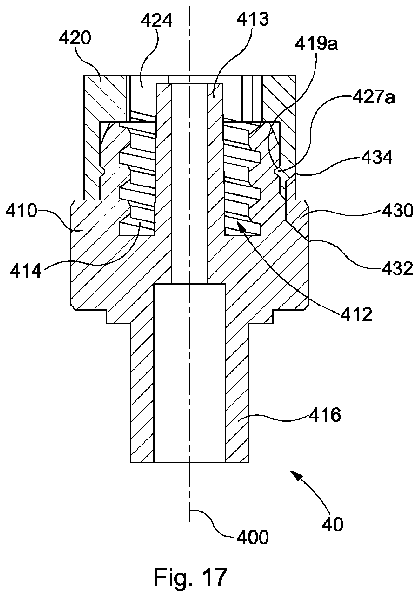

Figure 17 shows a cross-sectional view of a one-piece fluid line connector 40 according to another embodiment of the present invention. The one-piece fluid line connector 40 shares features with the one-piece fluid line connector 20 and the one-piece fluid line connector 30 described above and shown in Figures 3A to 4B, and 11 to 13C respectively, and such shared features are numbered using the same last two digits and a differing preceding digit. Differing or additional features of the one-piece fluid line connector 40 are described below. In certain embodiments, any or all of the differing or additional features of the one-piece fluid line connector 40 may be incorporated into the one-piece fluid line connector 20 described above.

The one-piece fluid connector 40 includes a cap 420 coupled to a main body 410 of the fluid line connector 40 by a living hinge 430. Like the one-piece fluid line connector 20 of Figures 3B and 4B, the one-piece fluid line connector 40 is shown in an aligned configuration, in which the cap 420 is aligned with a central axis 400 of the main body 410. Like the cap 320 of the one-piece fluid line connector 30, the cap 420 includes a through-hole 424 configured to receive a correspondingly-shaped female luer lock fitting. In the aligned configuration shown in Figure 13, the cap 420 is positioned adjacent to the beginning of a thread 414 of the male luer lock fitting 412.

The one-piece fluid line connector 40 of Figure 16 is distinguished from the one-piece fluid line connector 30 of Figure 11 in that the cap 420 includes a flange 427a configured to receive a receivable portion 419a of the main body 410 such that, when the cap 420 is received by the main body 410, the cap 420 is radially external of the main body 410. Whereas, in the one-piece fluid line connector 30 of Figure 11, the main body 310 includes a cap receiving portion 318 which receives the cap 320 such that, when the cap 320 is received by the main body 310, the cap receiving portion 318 is radially external of the cap 320.

In certain embodiments, the flange and the recess of the cap may be continuous or discontinuous around their respective circumferences, so long as a snap-fit fastening is achieved between the flange and the recess when the one-piece fluid line connector is in the aligned configuration. Preferably, the flange and the recess are continuous around their respective circumferences.

In certain embodiments, the cap may attach to the main body of the one-piece fluid line connector by any means such that the cap is fastenable to the main body to permit a non-standard female luer lock fitting to pass therethrough. For example, the cap could screw onto, form a magnetic connection with, or form a friction fit with the main body.

In certain embodiments, the conduit of the main body may be attached to the fluid feed container or a standardised male luer lock fitting by welding, gluing or any other suitable means.

In certain embodiments, the female conduit of the female luer lock fitting may be attached to the fluid feed line or a standardised female luer lock fitting by welding, gluing or any other suitable means.

In certain embodiments, the one-piece fluid line connector is an injection-moulded plastics material.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments.

## Claims

1. A one-piece fluid line connector (10; 20; 30) for connecting two fluid lines together to form a fluid connection comprising:
a main body (110; 210; 310) having a male luer lock fitting (112; 212; 312); and
a cap (120; 220; 320) coupled to the main body by a living hinge (130; 230; 330);
wherein the living hinge is configured such that the cap is moveable to an aligned position in which the cap is aligned with a central axis (100; 200; 300) of the male luer lock fitting of the main body, and
wherein the cap includes a through-hole (124; 224; 324) configured such that, in the aligned position, the shape of the through-hole permits a correspondingly-shaped female luer lock fitting (50; 90) to pass therethrough and engage the male luer lock fitting.

2. A one-piece fluid line connector (10; 20; 30) according to claim 1, wherein the male luer lock fitting (112; 212; 312) includes a thread (114; 214; 314) and a luer taper (113; 213; 313), the luer taper is configured to be received by a correspondingly-shaped female luer lock fitting (50; 90) and the thread is configured to receive one or more lugs (52; 92) of the correspondingly-shaped female luer lock fitting (50; 90).

3. A one-piece fluid line connector (30) according to claim 2, wherein the cap (320) has a guide portion (327), the guide portion being configured to determine a helical movement of at least one of the one or more lugs (92) between the cap and the thread (314) when the female luer lock fitting (90) is rotated in the cap, the at least one of the one or more lugs is adjacent the guide portion of the cap, and the cap is in the aligned position.

4. A one-piece fluid line connector (10; 20; 30) according to any preceding claim, wherein the through-hole (124; 224; 324) is configured to rotationally restrain at least one lug (52; 92) of a correspondingly-shaped female luer lock fitting (50; 90) in at least one axial position of the female luer lock fitting within the cap (120; 220; 320).

5. A one-piece fluid line connector (10; 20; 30) according to any preceding claim, wherein, in the aligned position, the cap (120; 220; 320) is fastenable to the main body (110; 210; 310) and optionally, wherein the cap is fastenable to the male luer lock fitting (112; 212; 312) of the main body.

6. A one-piece fluid line connector (10; 20; 30) according to claim 5, wherein the cap (120; 220; 230) is fastenable using a snap-fit connection and optionally wherein the cap is rotationally fixable relative to the main body (110; 210; 310).

7. A one-piece fluid line connector (10; 30) according to any preceding claim, wherein a portion of the main body (110; 310) is configured to receive the cap (120; 320) when the cap is in the aligned position, such that the portion of the main body is radially external to the cap.

8. A one-piece fluid line connector (20) according to any one of claims 1 to 6, wherein a portion of the cap (220) is configured to receive a portion of the main body (210) when the cap is in the aligned position, such that the portion of the cap is radially external to the main body.

9. A one-piece fluid line connector (10; 20; 30) according to any one of claims 2 to 8, wherein the through-hole (124; 224; 324) of the cap (120; 220; 320) is configured such that, when the cap is in the aligned position, as a lug (52; 92) of a correspondingly-shaped female luer lock (50; 90) fitting passes therethrough, the lug is aligned with an entrance of the thread (114; 214; 314) of the male luer lock fitting (112; 212; 312).

10. A one-piece fluid line connector (10; 20; 30) according to any preceding claim, wherein the through-hole (124; 224; 324) of the cap (120; 220; 320) has a variable radius and optionally wherein the through-hole of the cap comprises one or more adjacent sector with different radii.

11. A one-piece fluid line connector (10; 20; 30) according to any preceding claim, when dependent on claim 2, wherein when the cap (120; 220; 320) is in the aligned position, the cap is adjacent to the thread (114; 214; 314).

12. A one-piece fluid line connector (10; 20; 30) according to any preceding claim, wherein the main body (110; 210; 310) further comprises a conduit (116; 216; 316) configured to be attached to a fluid feed container to provide a fluid connection between the fluid feed container and the male luer lock fitting (112; 212; 312) of the main body.

13. A one-piece fluid line connector (10; 20) according to claim 2, further comprising a void (140; 210) when the cap (120; 220) is in the aligned position, the void positioned between the thread (114; 214) of the male luer lock fitting (112; 212) and the through-hole (124; 224) of the cap, the void configured to receive lugs (52) of a corresponding female luer lock fitting (50) and permit free rotation of the female luer lock fitting when the lugs are received by the void and optionally wherein the cap further comprises a stopper (125) configured to partially prevent counter-clockwise rotation of a corresponding female luer lock fitting when lugs of the corresponding female luer lock fitting are positioned within the void of the one-piece fluid line connector.

14. A one-piece fluid line connector (10; 20; 30) according to any preceding claim, further comprising indicator means configured to aid the user in aligning lugs (52; 92) of a female luer lock fitting (50; 90) with the through-hole (124; 224; 324) of the one-piece fluid line connector and optionally wherein the indicator means comprises a tactile indicator and optionally wherein the tactile indicator is positioned on the cap (120; 220; 320).

15. A fluid line assembly comprising:
a one-piece fluid line connector (10; 20; 30) according to any one of claims 2 to 14; and
a female luer lock fitting (50; 90) connectable to the one-piece fluid line connector, the female luer lock fitting comprising one or more lugs (52; 92) configured to be received by the thread (114; 214; 314) of the male luer lock fitting (112; 212; 312) of the one-piece fluid line connector;
wherein the female luer lock fitting is configured to connect with the one-piece fluid line connector such that a fluid may flow between the main body (110; 210; 310) of the one-piece fluid line connector and the female luer lock fitting.

## Patentansprüche

1. Einstückiger Fluidleitungsverbinder (10; 20; 30) zum Verbinden zweier Fluidleitungen miteinander, um eine Fluidverbindung zu bilden, umfassend:
einen Hauptkörper (110; 210; 310) mit einem männlichen Luer-Lock-Anschlussstück (112; 212; 312); und
eine Kappe (120; 220; 320), die durch ein Filmscharnier (130; 230; 330) mit dem Hauptkörper gekoppelt ist;
wobei das Filmscharnier so konfiguriert ist, dass die Kappe in eine ausgerichtete Position bewegbar ist, in der die Kappe auf eine Mittelachse (100; 200; 300) des männlichen Luer-Lock-Anschlussstücks des Hauptkörpers ausgerichtet ist, und
wobei die Kappe ein Durchgangsloch (124; 224; 324) enthält, das so konfiguriert ist, dass die Form des Durchgangslochs in der ausgerichteten Position es einem entsprechend geformten weiblichen Luer-Lock-Anschlussstück (50; 90) ermöglicht, durch dieses durchzutreten und den männlichen Luer-Lock-Anschluss in Eingriff zu nehmen.

2. Einstückiger Fluidleitungsverbinder (10; 20; 30) nach Anspruch 1, wobei das männliche Luer-Lock-Anschlussstück (112; 212; 312) ein Gewinde (114; 214; 314) und einen Luer-Konus (113; 213; 313) enthält, wobei der Luer-Konus dazu konfiguriert ist, von einem entsprechend geformten weiblichen Luer-Lock-Anschlussstück (50; 90) aufgenommen zu werden, und wobei das Gewinde dazu konfiguriert ist, einen oder mehrere Vorsprünge (52; 92) des entsprechend geformten weiblichen Luer-Lock-Anschlussstücks (50; 90) aufzunehmen.

3. Einstückiger Fluidleitungsverbinder (30) nach Anspruch 2, wobei die Kappe (320) einen Führungsabschnitt (327) aufweist, wobei der Führungsabschnitt dazu konfiguriert ist, eine spiralförmige Bewegung von mindestens einer des einen oder der mehreren Vorsprünge (92) zwischen der Kappe und dem Gewinde (314) zu bestimmen, wenn das weibliche Luer-Lock-Anschlussstück (90) in der Kappe gedreht wird, wobei der mindestens eine des einen oder der mehreren Vorsprünge an den Führungsabschnitt der Kappe angrenzt und die Kappe sich in der ausgerichteten Position befindet.

4. Einstückiger Fluidleitungsverbinder (10; 20; 30) nach einem vorhergehenden Anspruch, wobei das Durchgangsloch (124; 224; 324) dazu konfiguriert ist, mindestens einen Vorsprung (52; 92) eines entsprechend geformten weiblichen Luer-Lock-Anschlussstücks (50; 90) in mindestens einer axialen Position des weiblichen Luer-Lock-Anschlussstücks innerhalb der Kappe (120; 220; 320) rotatorisch zu halten.

5. Einstückiger Fluidleitungsverbinder (10; 20; 30) nach einem vorhergehenden Anspruch, wobei die Kappe (120; 220; 320) in der ausgerichteten Position an dem Hauptkörper (110; 210; 310) befestigbar ist und optional wobei die Kappe an dem männlichen Luer-Lock-Anschlussstück (112; 212; 312) des Hauptkörpers befestigbar ist.

6. Einstückiger Fluidleitungsverbinder (10; 20; 30) nach Anspruch 5, wobei die Kappe (120; 220; 230) unter Verwendung einer Schnappverbindung befestigbar ist und wobei optional die Kappe relativ zum Hauptkörper (110; 210; 310) rotatorisch fixierbar ist.

7. Einstückiger Fluidleitungsverbinder (10; 30) nach einem vorhergehenden Anspruch, wobei ein Abschnitt des Hauptkörpers (110; 310) dazu konfiguriert ist, die Kappe (120; 320) aufzunehmen, wenn sich die Kappe in der ausgerichteten Position befindet, so dass der Abschnitt des Hauptkörpers radial außerhalb der Kappe liegt.

8. Einstückiger Fluidleitungsverbinder (20) nach einem der Ansprüche 1 bis 6, wobei ein Abschnitt der Kappe (220) dazu konfiguriert ist, einen Abschnitt des Hauptkörpers (210) aufzunehmen, wenn sich die Kappe in der ausgerichteten Position befindet, so dass der Abschnitt der Kappe radial außerhalb des Hauptkörpers liegt.

9. Einstückiger Fluidleitungsverbinder (10; 20; 30) nach einem der Ansprüche 2 bis 8, wobei das Durchgangsloch (124; 224; 324) der Kappe (120; 220; 320) so konfiguriert ist, dass, wenn sich die Kappe in der ausgerichteten Position befindet, wenn ein Vorsprung (52; 92) eines entsprechend geformten weiblichen Luer-Lock-Anschlussstücks (50; 90) durch es hindurchtritt, der Vorsprung auf einen Eingang des Gewindes (114; 214; 314) des männlichen Luer-Lock-Anschlussstücks (112; 212; 312) ausgerichtet ist.

10. Einstückiger Fluidleitungsverbinder (10; 20; 30) nach einem vorhergehenden Anspruch, wobei das Durchgangsloch (124; 224; 324) der Kappe (120; 220; 320) einen variablen Radius aufweist und wobei optional das Durchgangsloch der Kappe einen oder mehrere angrenzende Sektoren mit unterschiedlichen Radien umfasst.

11. Einstückiger Fluidleitungsverbinder (10; 20; 30) nach einem vorhergehenden Anspruch, wenn abhängig von Anspruch 2, wobei, wenn sich die Kappe (120; 220; 320) in der ausgerichteten Position befindet, die Kappe an das Gewinde (114; 214; 314) angrenzt.

12. Einstückiger Fluidleitungsverbinder (10; 20; 30) nach einem vorhergehenden Anspruch, wobei der Hauptkörper (110; 210; 310) ferner eine Röhre (116; 216; 316) umfasst, die dazu konfiguriert ist, an einem Fluidzufuhrbehälter angebracht zu werden, um eine Fluidverbindung zwischen dem Fluidzufuhrbehälter und dem männlichen Luer-Lock-Anschlussstück (112; 212; 312) des Hauptkörpers bereitzustellen.

13. Einstückiger Fluidleitungsverbinder (10; 20) nach Anspruch 2, ferner umfassend einen Hohlraum (140; 210), wenn sich die Kappe (120; 220) in der ausgerichteten Position befindet, wobei der Hohlraum zwischen dem Gewinde (114; 214) des männlichen Luer-Lock-Anschlussstücks (112; 212) und dem Durchgangsloch (124; 224) der Kappe positioniert ist, wobei der Hohlraum dazu konfiguriert ist, Vorsprünge (52) eines entsprechenden weiblichen Luer-Lock-Anschlussstücks (50) aufzunehmen und eine freie Drehung des weiblichen Luer-Lock-Anschlussstücks zu ermöglichen, wenn die Vorsprünge von dem Hohlraum aufgenommen sind, und wobei optional die Kappe ferner einen Anschlag (125) umfasst, der dazu konfiguriert ist, teilweise eine Drehung gegen den Uhrzeigersinn eines entsprechenden weiblichen Luer-Lock-Anschlussstücks zu verhindern, wenn Vorsprünge des entsprechenden weiblichen Luer-Lock-Anschlussstücks innerhalb des Hohlraums des einstückigen Fluidleitungsverbinders positioniert sind.

14. Einstückiger Fluidleitungsverbinder (10; 20; 30) nach einem vorhergehenden Anspruch, ferner umfassend ein Anzeigemittel, das dazu konfiguriert ist, dem Benutzer beim Ausrichten von Vorsprüngen (52; 92) eines weiblichen Luer-Lock-Anschlussstücks (50; 90) auf das Durchgangsloch (124; 224; 324) des einstückigen Fluidleitungsverbinders zu helfen, und optional wobei das Anzeigemittel ein tastbares Anzeigemittel umfasst und optional wobei das tastbare Anzeigemittel auf der Kappe (120; 220; 320) positioniert ist.

15. Fluidleitungsbaugruppe, umfassend:
einen einstückigen Fluidleitungsverbinder (10; 20; 30) nach einem der Ansprüche 2 bis 14; und
ein weibliches Luer-Lock-Anschlussstück (50; 90), das mit dem einstückigen Fluidleitungsverbinder verbindbar ist, wobei das weibliche Luer-Lock-Anschlussstück einen oder mehrere Vorsprünge (52; 92) umfasst, die dazu konfiguriert sind, von dem Gewinde (114; 214; 314) des männlichen Luer-Lock-Anschlussstücks (112; 212; 312) des einstückigen Fluidleitungsverbinders aufgenommen werden;
wobei das weibliche Luer-Lock-Anschlussstück dazu konfiguriert ist, sich mit dem einstückigen Fluidleitungsverbinder so zu verbinden, dass ein Fluid zwischen dem Hauptkörper (110; 210; 310) des einstückigen Fluidleitungsverbinders und dem weiblichen Luer-Lock-Anschlussstück fließen kann.

## Revendications

1. Raccord de conduite de fluide en une seule pièce (10 ; 20 ; 30) destiné à connecter deux conduites de fluide ensemble pour former un raccordement fluidique comprenant :
un corps principal (110; 210; 310) possédant un raccord Luer-Lock mâle (112; 212; 312); et
un capuchon (120 ; 220 ; 320) couplé au corps principal par une charnière souple (130 ; 230 ; 330) ;
ladite charnière souple étant est conçue de sorte que le capuchon soit mobile vers une position alignée dans laquelle le capuchon est aligné avec un axe central (100 ; 200 ; 300) du raccord Luer-Lock mâle du corps principal, et
ledit capuchon comprenant un trou traversant (124 ; 224 ; 324) conçu de sorte que, dans la position alignée, la forme du trou traversant permette au raccord Luer-Lock femelle de forme correspondante (50 ; 90) de passer à travers celui-ci et de se mettre en prise avec le raccord Luer-Lock mâle.

2. Raccord de conduite de fluide en une seule pièce (10 ; 20 ; 30) selon la revendication 1, ledit raccord Luer-Lock mâle (112 ; 212 ; 312) comprenant un filet (114 ; 214 ; 314) et un cône Luer (113 ; 213 ; 313), ledit cône Luer étant conçu pour être reçu par un raccord Luer-Lock femelle de forme correspondante (50 ; 90) et ledit filet étant conçu pour recevoir un ou plusieurs ergots (52 ; 92) du raccord Luer-Lock femelle de forme correspondante (50 ; 90).

3. Raccord de conduite de fluide en une seule pièce (30) selon la revendication 2, ledit capuchon (320) comportant une partie de guidage (327), la partie de guidage étant conçue pour déterminer un mouvement hélicoïdal d'au moins l'un du ou des ergots (92) entre le capuchon et le filet (314) lorsque le raccord Luer-Lock femelle (90) est tourné dans le capuchon, ledit au moins un du ou des ergots étant adjacents à la partie de guidage du capuchon, et ledit capuchon étant dans la position alignée.

4. Raccord de conduite de fluide en une seule pièce (10 ; 20 ; 30) selon une quelconque revendication précédente, ledit trou traversant (124 ; 224 ; 324) étant conçu pour retenir en rotation au moins un ergot (52 ; 92) d'un raccord Luer-Lock femelle de forme correspondante (50 ; 90) dans au moins une position axiale du raccord Luer-Lock femelle à l'intérieur du capuchon (120 ; 220 ; 320).

5. Raccord de conduite de fluide en une seule pièce (10 ; 20 ; 30) selon une quelconque revendication précédente, dans la position alignée, ledit capuchon (120 ; 220 ; 320) pouvant être fixé au corps principal (110 ; 210 ; 310) et éventuellement, ledit capuchon pouvant être fixé au raccord Luer-Lock mâle (112 ; 212 ; 312) du corps principal.

6. Raccord de conduite de fluide en une seule pièce (10 ; 20 ; 30) selon la revendication 5, ledit capuchon (120 ; 220 ; 230) pouvant être fixé à l'aide d'un raccordement à encliquetage et éventuellement ledit capuchon pouvant être fixé en rotation par rapport au corps principal. (110 ; 210 ; 310).

7. Raccord de conduite de fluide en une seule pièce (10 ; 30) selon une quelconque revendication précédente, une partie du corps principal (110 ; 310) étant conçue pour recevoir le capuchon (120 ; 320) lorsque le capuchon est dans la position alignée, de sorte que la partie du corps principal soit radialement extérieure au capuchon.

8. Raccord de conduite de fluide en une seule pièce (20) selon l'une quelconque des revendications 1 à 6, une partie du capuchon (220) étant conçue pour recevoir une partie du corps principal (210) lorsque le capuchon est dans la position alignée, de sorte que la partie du capuchon soit radialement extérieure au corps principal.

9. Raccord de conduite de fluide en une seule pièce (10 ; 20 ; 30) selon l'une quelconque des revendications 2 à 8, ledit trou traversant (124 ; 224 ; 324) du capuchon (120 ; 220 ; 320) étant conçu de sorte que, lorsque le capuchon est dans la position alignée, tandis qu'un ergot (52 ; 92) d'un raccord Luer-Lock femelle de forme correspondante (50 ; 90) passe à travers lui, l'ergot soit aligné avec une entrée du filet (114 ; 214 ; 314) du raccord Luer-Lock mâle (112 ; 212 ; 312).

10. Raccord de conduite de fluide en une seule pièce (10 ; 20 ; 30) selon une quelconque revendication précédente, ledit trou traversant (124 ; 224 ; 324) du capuchon (120 ; 220 ; 320) possédant un rayon variable et éventuellement ledit le trou traversant du capuchon comprenant un ou plusieurs secteurs adjacents avec des rayons différents.

11. Raccord de conduite de fluide en une seule pièce (10 ; 20 ; 30) selon une quelconque revendication précédente, lorsqu'elle dépend de la revendication 2, lorsque le capuchon (120 ; 220 ; 320) est dans la position alignée, ledit capuchon étant adjacent au filet (114; 214; 314).

12. Raccord de conduite de fluide en une seule pièce (10 ; 20 ; 30) selon une quelconque revendication précédente, ledit corps principal (110 ; 210 ; 310) comprenant en outre un conduit (116 ; 216 ; 316) conçu pour être fixé à un récipient d'alimentation en fluide pour fournir un raccordement fluidique entre le récipient d'alimentation en fluide et le raccord Luer-Lock mâle (112 ; 212 ; 312) du corps principal.

13. Raccord de conduite de fluide en une seule pièce (10 ; 20) selon la revendication 2, comprenant en outre un vide (140 ; 210) lorsque le capuchon (120 ; 220) est dans la position alignée, le vide étant positionné entre le filet (114 ; 214) du raccord Luer-Lock mâle (112 ; 212) et du trou traversant (124 ; 224) du capuchon, le vide étant conçu pour recevoir des ergots (52) d'un raccord Luer-Lock femelle correspondant (50) et permettre la libre rotation du raccord Luer-Lock femelle lorsque les ergots sont reçus par le vide et éventuellement ledit capuchon comprenant en outre une butée (125) conçue pour empêcher partiellement la rotation dans le sens antihoraire d'un raccord Luer-Lock femelle correspondant lorsque les ergots du raccord Luer-Lock femelle correspondant sont positionnés à l'intérieur du vide du raccord de conduite de fluide en une seule pièce.

14. Raccord de conduite de fluide en une seule pièce (10 ; 20 ; 30) selon une quelconque revendication précédente, comprenant en outre un moyen indicateur conçu pour aider l'utilisateur à aligner les ergots (52 ; 92) d'un raccord Luer-Lock femelle (50 ; 90) avec le trou traversant (124 ; 224 ; 324) du raccord de ligne de fluide en une seule pièce et éventuellement ledit moyen indicateur comprenant un indicateur tactile et éventuellement ledit indicateur tactile étant positionné sur le capuchon (120 ; 220 ; 320).

15. Ensemble conduite de fluide comprenant :
un raccord de conduite de fluide en une seule pièce (10 ; 20 ; 30) selon l'une quelconque des revendications 2 à 14 ; et un raccord Luer-Lock femelle (50 ; 90) pouvant être raccordé au raccord de conduite de fluide en une seule pièce, le raccord Luer-Lock femelle comprenant un ou plusieurs ergots (52 ; 92) conçus pour être reçus par le filet (114 ; 214 ; 314) du raccord Luer-Lock mâle (112 ; 212 ; 312) du raccord en une seule pièce de conduite de fluide ;
ledit raccord Luer-Lock femelle étant conçu pour se raccorder au raccord de conduite de fluide en une seule pièce de sorte qu'un fluide puisse s'écouler entre le corps principal (110 ; 210 ; 310) du raccord de conduite de fluide en une seule pièce et le raccord Luer-Lock femelle.
